# EUROPEAN PATENT APPLICATION

(11) **EP 3 944 852 A1**
(43) Date of publication of application: **02.02.2022**
(21) Application number: 20188266.9
(22) Date of filing: 28.07.2020
(51) Int. Cl.: A61K 8/34, A61K 8/49, A61Q 5/00

(54) **USE OF A BOOSTER TO ENHANCE THE ANTI-DANDRUFF OR PRESERVATIVE ACTIVITY OF PIROCTONE OLAMINE**

(71) Applicant: Clariant International Ltd, 4132 Muttenz (CH)
(72) Inventor: SCHAAL, Petra, 65926 Frankfurt am Main (DE); GROHMANN, Peter, 65926 Frankfurt am Main (DE); SIEFER, Beate, 65926 Frankfurt am Main (DE)
(74) Representative: Kampen, Daniela

(57) **Abstract**

The invention relates to the use of a booster to enhance the anti-dandruff or preservative activity of piroctone olamine, as well as to blends and cosmetic compositions comprising piroctone olamine and a booster.

## Description

The invention relates to the use of a booster to enhance the anti-dandruff or preservative activity of piroctone olamine, as well as to blends and cosmetic compositions comprising piroctone olamine and a booster.

Piroctone olamine (Octopirox^{®}) is an effective, nontoxic anti-dandruff agent. It is particularly suitable for the manufacture of anti-dandruff shampoos and hair care products such as hair tonics and cream rinses. Microorganisms such as Malassezia furfur produce enzymes which decompose fats into their respective fatty acids. These and other products of decomposition, such as lipo-peroxides, irritate the scalp. As a result, mitosis and production of keratinocytes increases, leading to desquamation and parakeratosis. Piroctone olamine (Octopirox^{®}) controls the growth of microorganisms effectively and directly targets the cause of dandruff.

Preservatives are added to most personal care products today in order to inhibit growth of bacteria and fungi. Piroctone olamine (Octopirox^{®}) has been successfully used for over 30 years because of its good compatibility, antimicrobial functionality, and long shelf life. It is generally considered safe, non-irritating, and non-allergenic.

GB1440975, EP0158481, and WO2006/081969, among others, describe the use of piroctone olamine (Octopirox^{®}) as an anti-dandruff agent and/or as a preservative.

However, there is a desire for improved anti-dandruff and/or preservative systems. In particular, there is a desire for improved efficacy of such systems, which could allow for reduced levels of the anti-dandruff agent and/or preservative in the cosmetic end product. Consumers have generally become more conscious about the ingredients used in cosmetic products. Among others, they desire reduced levels of antimicrobial agents. Furthermore, there may be regulatory restrictions. There is also a desire for sustainable and environmentally friendly anti-dandruff and/or preservative systems.

It has now surprisingly been found that alcohols having 2 to 5 hydroxy groups or plant extracts can boost the activity of piroctone olamine. Combinations of piroctone olamine and such boosters even have a synergistic effect.

Accordingly, the invention relates to the use of a booster to enhance the anti-dandruff activity of piroctone olamine or to enhance the preservative activity of piroctone olamine, wherein the booster is selected from alcohols having 2 to 5 hydroxy groups and plant extracts.

In preferred embodiments, the invention relates to the use of a booster to enhance the anti-dandruff activity of piroctone olamine.

In preferred embodiments, the invention relates to the use of a booster to enhance the preservative activity of piroctone olamine.

Particularly preferably, piroctone olamine and the booster are part of a cosmetic composition. Preferably, the cosmetic composition is a shampoo composition or hair conditioner composition. More preferably, the cosmetic composition is a shampoo composition, particularly preferably an anti-dandruff shampoo composition. Also more preferably, the cosmetic composition of the invention is a hair conditioner composition.

Preferably, the dandruff is caused by dandruff-causing organisms, more preferably Malassezia species, even more preferably Malassezia furfur and/or Malassezia globosa, particularly preferably Malassezia furfur, also particularly preferably Malassezia globosa.

Piroctone olamine is also referred to as piroctone ethanolamine or 1-hydroxy-4-methyl-6-(2,4,4-trimethyl)-pentyl-2(1 H)-pyridone, 2-aminoethanol salt.

One or more boosters may be used. Preferably, 1 to 3 boosters are used, more preferably 1 or 2 boosters, particularly preferably 1 booster, also particularly preferably 2 boosters.

In preferred embodiments, the booster is selected from alcohols having 2 to 5 hydroxy groups.

Particularly preferred alcohols are alcohols having 2 hydroxy groups. Alcohols having 2 hydroxy groups are herein also referred to as diols.

Preferred diols are diols having 2 to 20 carbon atoms, preferably 3 to 15 carbon atoms, more preferably 4 to 12 carbon atoms, particularly preferably 6 to 10 carbon atoms, for example 8 carbon atoms.

More preferred diols are ethylhexylglycerin, caprylyl glycol, caprylyl glyceryl ether, 1,3-octanediol, 1,2-hexanediol, pentylene glycol, 1,2-decanediol (decylene glycol), 4-n-butyl resorcinol, or mixtures thereof. Ethylhexylglycerin is commercially available from Clariant (Velsan^{®} EHG). Caprylyl glyceryl ether is commercially available from Clariant (Velsan^{®} CGE).

Even more preferred diols are ethylhexylglycerin, caprylyl glycol, caprylyl glyceryl ether, 1,3-octanediol, or mixtures thereof. Even more preferred diols are ethylhexylglycerin, caprylyl glycol, or mixtures thereof. A particularly preferred diol is ethylhexylglycerin.

Also preferred alcohols are alcohols having 3 hydroxy groups. Alcohols having 3 hydroxy groups are herein also referred to as triols.

Preferred triols are triols having 3 to 30 carbon atoms, preferably 6 to 25 carbon atoms, more preferably 12 to 25 carbon atoms, particularly preferably 19 to 21 carbon atoms.

A more preferred triol is sorbitan caprylate. Sorbitan caprylate is commercially available from Clariant (Velsan^{®} SC).

Also more preferred triols are triols of formula (I), 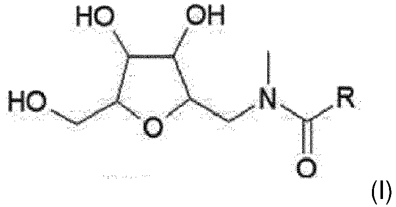 wherein R is selected from saturated or unsaturated hydrocarbon chains having 5 to 23 carbon atoms. Preferably, R in formula (I) is selected from saturated or unsaturated hydrocarbon chains having 7 to 17 carbon atoms. More preferably, R in formula (I) is selected from saturated or unsaturated hydrocarbon chains having 7 to 13 carbon atoms. Even more preferably, R in formula (I) is -(CH₂)₆CH₃, -(CH₂)₈CH₃, -(CH₂)₁₀CH₃, -(CH₂)₁₂CH₃, or mixtures thereof. Also even more preferably, the R-C=O residue in formula (I) is derived from cocoyl fatty acids, 9-decenoic acid, 9-dodecenoic acid, or mixtures thereof. Particularly preferably, R in formula (I) is - (CH₂)₁₀CH₃, -(CH₂)₁₂CH₃, or mixtures thereof. Also particularly preferably, the R-C=O residue in formula (I) is derived from 9-decenoic acid, 9-dodecenoic acid, or mixtures thereof. Capryloyl/caproyl anhydro methyl glucamide is commercially available from Clariant (Velsan^{®} Flex).

Also preferred alcohols are alcohols having 4 hydroxy groups. Examples of alcohols having 4 hydroxy groups are glucosamine (2-amino-2-deoxy-glucose), N-methylglucosamine, N,N-dimethylglucosamine, or mixtures thereof.

Also preferred alcohols are alcohols having 5 hydroxy groups. Preferred alcohols having 5 hydroxy groups are N-methyl-N-acylglucamines of formula (II) 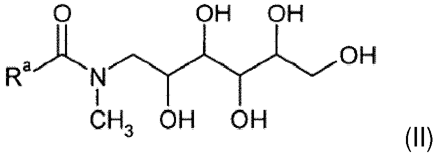 wherein R^{a} is selected from saturated or unsaturated hydrocarbon chains having 5 to 23 carbon atoms. Preferably, R^{a} in formula (II) is selected from saturated or unsaturated hydrocarbon chains having 7 to 17 carbon atoms. Also preferably, the R^{a}-C=O residue in formula (II) is derived from caprylic acid, capric acid, lauric acid, myristic acid, palmitic acid, stearic acid, oleic acid, linoleic acid, linolenic acid, cocoyl fatty acids, or mixtures thereof. Also preferably, the R^{a}-C=O residue in formula (II) is derived from 9-decenoic acid, 9-dodecenoic acid, or mixtures thereof.

Particularly preferred N-methyl-N-acylglucamines of formula (II) are capryloyl/caproyl methyl glucamide, lauroyl/myristoyl methyl glucamide, cocoyl methyl glucamide, oleyl methyl glucamide, or mixtures thereof. Such N-methyl-N-acylglucamines are commercially available from Clariant (GlucoTain^{®} Clear, GlucoTain^{®} Plus, GlucoTain^{®} Flex, GlucoTain^{®} Care, GlucoTain^{®} Sense).

Also particularly preferred N-methyl-N-acylglucamines of formula (II) are N-9-decenoyl-N-methylglucamine, N-9-dodecenoyl-N-methylglucamine, or mixtures thereof.

Further examples of alcohols are N-methylglucamine, N,N-dimethylglucamine, or mixtures thereof. Such glucamines are commercially available from Clariant (e.g. NeutroTain^{™} DMG).

In preferred embodiments, the booster is selected from plant extracts.

Preferred plant extracts are peel extracts, pericarp extracts, fruit extracts, leaf extracts, flower extracts, stem extracts, seed extracts, or mixtures thereof. More preferred plant extracts are peel extracts, leaf extracts, or mixtures thereof.

Examples of preferred plant extracts are Garcinia Mangostana peel extract, Punica Granatum pericarp extract, Capsicum Frutescens fruit extract, Citrus Hystrix leaf extract, Centella Asiatica flower/leaf/stem extract, Saccharum Officinarum extract, Psidium Guajava fruit extract, Ocimum Basilicum leaf extract, Orthosiphon Stamineus leaf extract, Phyllanthus Niruri extract, Guazuma Ulmifolia leaf extract, Phyllantus Niruri extract, Centella Asiatica flower/leaf/stem extract, Glycine Max seed extract, Camellia Sinensis leaf extract, or mixtures thereof. Examples of more preferred plant extracts are Garcinia Mangostana peel extract, Orthosiphon Stamineus leaf extract, or mixtures thereof. The CAS Registry Number of Garcinia Mangostana peel extract is 90045-25-3. The CAS Registry Number of Orthosiphon Stamineus leaf extract is 84012-29-3.

Such plant extracts are commercially available from Clariant (Plantasens^{®} Berto STEENOBRIGHT, Plantasens^{®} Berto POMEOBRIGHT, Plantasens^{®} Berto CAPSIBRIGHT, Plantasens^{®} Berto HYSTOBRIGHT, Plantasens^{®} Berto GOTUBRIGHT, Plantasens^{®} Berto CANEMOIST, Plantasens^{®} Berto GUAVOMOIST, Plantasens^{®} Berto OCIMOIST, Plantasens^{®} Berto ORTHOMOIST, Plantasens^{®} Berto PHYLLANOXI, Plantasens^{®} Berto OXI COMPLEX, Plantasens^{®} Berto JAVA COMPLEX).

The plant extracts used in the invention can be prepared by methods known in the art. For example, the plant extracts can be prepared by extracting plants or parts thereof using a suitable solvent. Suitable solvents are, for example, water, alcohols, or mixtures thereof. Preferred solvents are water, ethanol, glycerin, or mixtures thereof. Particularly preferred solvents are water / ethanol mixtures or water / glycerin mixtures. The plants or parts thereof can, for example, be extracted at room temperature (20-25 °C) or at elevated temperatures (e.g. 30-80 °C or 40-70 °C).

In particular embodiments, a booster selected from alcohols having 2 to 5 hydroxy groups and a booster selected from plant extracts are used.

Advantageously, the boosters are sustainable and environmentally friendly.

Preferably, the weight ratio of piroctone olamine to the booster is from 0.02 to 2.4, more preferably from 0.05 to 1.5, particularly preferably from 0.1 to 1.2.

More preferably, the weight ratio of piroctone olamine to the booster is from 0.02 to 0.7, more preferably from 0.05 to 0.5, particularly preferably from 0.1 to 0.4.

Also more preferably, the weight ratio of piroctone olamine to the booster is from 0.25 to 2.4, more preferably from 0.5 to 1.5, particularly preferably from 0.8 to 1.2.

Piroctone olamine and the booster may be used separately or in the form of a blend. In preferred embodiments, piroctone olamine and the booster are used separately. In also preferred embodiments, piroctone olamine and the booster are used in the form of a blend. In particular embodiments, piroctone olamine and the booster are used in the form of a blend of the invention.

The invention also relates to a blend comprising piroctone olamine and a booster selected from alcohols having 2 to 5 hydroxy groups and plant extracts.

The blend of the invention may comprise one or more boosters. Preferably, the blend of the invention comprises 1 to 3 boosters, more preferably 1 or 2 boosters, particularly preferably 1 booster, also particularly preferably 2 boosters.

In preferred embodiments, the booster is selected from alcohols having 2 to 5 hydroxy groups. Preferred alcohols having 2 to 5 hydroxy groups are described further above.

In preferred embodiments, the booster is selected from plant extracts. Preferred plant extracts are described further above.

In particular embodiments, the blend of the invention comprises a booster selected from alcohols having 2 to 5 hydroxy groups and a booster selected from plant extracts.

In preferred embodiments, the blend of the invention comprises from 2 to 70 wt.-%, preferably from 5 to 60 wt.-%, particularly preferably from 10 to 55 wt.-% of piroctone olamine, based on the total weight of the blend.

In more preferred embodiments, the blend of the invention comprises from 2 to 40 wt.-%, preferably from 5 to 30 wt.-%, particularly preferably from 10 to 25 wt.-% of piroctone olamine, based on the total weight of the blend.

In also more preferred embodiments, the blend of the invention comprises from 20 to 70 wt.-%, preferably from 35 to 60 wt.-%, particularly preferably from 45 to 55 wt.-% of piroctone olamine, based on the total weight of the blend.

In preferred embodiments, the blend of the invention comprises from 30 to 98 wt.-%, preferably from 40 to 95 wt.-%, particularly preferably from 45 to 90 wt.-% of the booster, based on the total weight of the blend.

In more preferred embodiments, the blend of the invention comprises from 60 to 98 wt.-%, preferably from 70 to 95 wt.-%, particularly preferably from 75 to 90 wt.-% of the booster, based on the total weight of the blend.

In also more preferred embodiments, the blend of the invention comprises from 30 to 80 wt.-%, preferably from 40 to 65 wt.-%, particularly preferably from 45 to 55 wt.-% of the booster, based on the total weight of the blend.

In preferred embodiments, the blend of the invention comprises
from 2 to 70 wt.-%, preferably from 5 to 60 wt.-%, particularly preferably from 10 to 55 wt.-% of piroctone olamine, based on the total weight of the blend, and
from 30 to 98 wt.-%, preferably from 40 to 95 wt.-%, particularly preferably from 45 to 90 wt.-% of the booster, based on the total weight of the blend.

In more preferred embodiments, the blend of the invention comprises
from 2 to 40 wt.-%, preferably from 5 to 30 wt.-%, particularly preferably from 10 to 25 wt.-% of piroctone olamine, based on the total weight of the blend, and
from 60 to 98 wt.-%, preferably from 70 to 95 wt.-%, particularly preferably from 75 to 90 wt.-% of the booster, based on the total weight of the blend.

In also more preferred embodiments, the blend of the invention comprises
from 20 to 70 wt.-%, preferably from 35 to 60 wt.-%, particularly preferably from 45 to 55 wt.-% of piroctone olamine, based on the total weight of the blend, and
from 30 to 80 wt.-%, preferably from 40 to 65 wt.-%, particularly preferably from 45 to 55 wt.-% of the booster, based on the total weight of the blend.

Preferably, the weight ratio of piroctone olamine to the booster is from 0.02 to 2.4, more preferably from 0.05 to 1.5, particularly preferably from 0.1 to 1.2.

More preferably, the weight ratio of piroctone olamine to the booster is from 0.02 to 0.7, more preferably from 0.05 to 0.5, particularly preferably from 0.1 to 0.4.

Also more preferably, the weight ratio of piroctone olamine to the booster is from 0.25 to 2.4, more preferably from 0.5 to 1.5, particularly preferably from 0.8 to 1.2.

Preferably, the sum of the amounts of piroctone olamine and the booster in the blend of the invention represents at least 20 wt.-%, more preferably at least 40 wt.-%, more preferably at least 50 wt.-%, more preferably at least 60 wt.-%, even more preferably at least 70 wt.-%, even more preferably at least 80 wt.-%, even more preferably at least 90 wt.-%, even more preferably at least 95 wt.-%, particularly preferably at least 98 wt.-%, based on the total weight of the blend.

In particular embodiments, the blend of the invention essentially consists of piroctone olamine and the booster. In particular embodiments, the blend of the invention consists of piroctone olamine and the booster.

Optionally, the blend of the invention comprises one or more additives (A).

Preferred additives (A) are solvents, aromatic sulfonates, or mixtures thereof.

More preferred additives (A) are solvents. Preferred solvents are water, alcohols, or mixtures thereof. More preferred solvents are glycerin, water, ethanol, propanol, isopropanol, ethylene glycol, propylene glycol, or mixtures thereof. Particularly preferred solvents are glycerin, water, ethanol, propylene glycol, or mixtures thereof.

Solvents are useful for providing the blend of the invention in liquid form. Advantageously, the above solvents are cosmetically acceptable. Water is particularly useful for economic reasons.

Also more preferred additives (A) are aromatic sulfonates. Preferred aromatic sulfonates are xylenesulfonates, cumenesulfonates, or mixtures thereof. Examples of preferred aromatic sulfonates are sodium xylenesulfonate, potassium xylenesulfonate, ammonium xylenesulfonate, sodium cumenesulfonate, potassium cumenesulfonate, ammonium cumenesulfonate, or mixtures thereof. Particularly preferred aromatic sulfonates are sodium xylenesulfonate, sodium cumenesulfonate, or mixtures thereof. Aromatic sulfonates are useful as solubilizing agents or hydrotropes.

Optionally, the blend of the invention may comprise further additives selected from the group consisting of oily substances, emulsifiers, coemulsifiers, cationic polymers, film formers, superfatting agents, stabilizers, preservatives, pearlizing agents, dyes, pigments, fragrances, opacifiers, functional acids, protein derivatives such as gelatin, collagen hydrolysates, natural or synthetic polypeptides, egg yolk, lecithin, lanolin, lanolin derivatives, fatty alcohols, silicones, carriers, and mixtures thereof. Optionally, the blend of the invention may comprise further additives selected from the group consisting of viscosity modifiers, dyes, pigments, pearlescent aids, thickeners, foam boosters, surfactants, co-surfactants, pH adjusting agents, perfumes, preservatives, proteins, sunscreens, UV absorbers, vitamins, niacinamide, and mixtures thereof.

In preferred embodiments, the blend of the invention comprises from 1 to 68 wt.-%, preferably from 3 to 55 wt.-%, particularly preferably from 5 to 50 wt.-% of one or more additives (A), based on the total weight of the blend.

In more preferred embodiments, the blend of the invention comprises from 1 to 38 wt.-%, preferably from 3 to 25 wt.-%, particularly preferably from 5 to 20 wt.-% of one or more additives (A), based on the total weight of the blend.

In also more preferred embodiments, the blend of the invention comprises from 1 to 50 wt.-%, preferably from 3 to 25 wt.-%, particularly preferably from 5 to 20 wt.-% of one or more additives (A), based on the total weight of the blend.

In preferred embodiments, the blend of the invention comprises
from 2 to 70 wt.-%, preferably from 5 to 60 wt.-%, particularly preferably from 10 to 55 wt.-% of piroctone olamine, based on the total weight of the blend,
from 30 to 98 wt.-%, preferably from 40 to 95 wt.-%, particularly preferably from 40 to 85 wt.-% of the booster, based on the total weight of the blend, and
from 1 to 68 wt.-%, preferably from 3 to 55 wt.-%, particularly preferably from 5 to 50 wt.-% of one or more additives (A), based on the total weight of the blend.

In more preferred embodiments, the blend of the invention comprises
from 2 to 40 wt.-%, preferably from 5 to 30 wt.-%, particularly preferably from 10 to 25 wt.-% of piroctone olamine, based on the total weight of the blend,
from 60 to 98 wt.-%, preferably from 70 to 95 wt.-%, particularly preferably from 70 to 85 wt.-% of the booster, based on the total weight of the blend, and
from 1 to 38 wt.-%, preferably from 3 to 25 wt.-%, particularly preferably from 5 to 20 wt.-% of one or more additives (A), based on the total weight of the blend.

In also more preferred embodiments, the blend of the invention comprises
from 20 to 70 wt.-%, preferably from 35 to 60 wt.-%, particularly preferably from 40 to 50 wt.-% of piroctone olamine, based on the total weight of the blend,
from 30 to 80 wt.-%, preferably from 40 to 65 wt.-%, particularly preferably from 40 to 50 wt.-% of the booster, based on the total weight of the blend, and
from 1 to 50 wt.-%, preferably from 3 to 25 wt.-%, particularly preferably from 5 to 20 wt.-% of one or more additives (A), based on the total weight of the blend.

In preferred embodiments, the blend of the invention is liquid. In also preferred embodiments, the blend of the invention is in solid form. For example, the blend of the invention can be in the form of a powder or granules.

The blend of the invention is particularly useful for anti-dandruff applications and/or for preserving cosmetic compositions.

Preferably, the blend of the invention is an anti-dandruff blend and/or a preservative blend. Particularly preferably, the blend of the invention is an anti-dandruff blend. Also particularly preferably, the blend of the invention is a preservative blend.

Preferably, the blend of the invention is used as an anti-dandruff blend and/or a preservative blend. Particularly preferably, the blend of the invention is used as an anti-dandruff blend. Also particularly preferably, the blend of the invention is used as a preservative blend.

The invention also relates to the use of the blend of the invention as an anti-dandruff blend. The invention also relates to the use of the blend of the invention as a preservative blend. The invention also relates to the use of the blend of the invention in a cosmetic composition, preferably a shampoo composition.

The blend of the invention can be prepared by methods known in the art. For example, the blend of the invention can be prepared by mixing its ingredients.

The blend of the invention may be used to prepare the cosmetic composition of the invention.

The invention also relates to a cosmetic composition comprising piroctone olamine and a booster selected from alcohols having 2 to 5 hydroxy groups and plant extracts.

The cosmetic composition of the invention may comprise one or more boosters. Preferably, the cosmetic composition of the invention comprises 1 to 3 boosters, more preferably 1 or 2 boosters, particularly preferably 1 booster, also particularly preferably 2 boosters.

In preferred embodiments, the booster is selected from alcohols having 2 to 5 hydroxy groups. Preferred alcohols having 2 to 5 hydroxy groups are described further above.

In preferred embodiments, the booster is selected from plant extracts. Preferred plant extracts are described further above.

In particular embodiments, the cosmetic composition of the invention comprises a booster selected from alcohols having 2 to 5 hydroxy groups and a booster selected from plant extracts.

In preferred embodiments, the cosmetic composition of the invention comprises from 0.02 to 7 wt.-%, preferably from 0.05 to 5 wt.-%, more preferably from 0.1 to 2.0 wt.-%, even more preferably from 0.1 to 1.0 wt.-%, particularly preferably from 0.1 to 0.5 wt.-% of piroctone olamine, based on the total weight of the cosmetic composition.

In preferred embodiments, the cosmetic composition of the invention comprises from 0.02 to 7 wt.-%, preferably from 0.05 to 5 wt.-%, more preferably from 0.1 to 2.0 wt.-%, even more preferably from 0.1 to 1.0 wt.-%, particularly preferably from 0.1 to 0.5 wt.-% of the booster, based on the total weight of the cosmetic composition.

In more preferred embodiments, the cosmetic composition of the invention comprises
from 0.02 to 7 wt.-%, preferably from 0.05 to 5 wt.-%, more preferably from 0.1 to 2.0 wt.-%, even more preferably from 0.1 to 1.0 wt.-%, particularly preferably from 0.1 to 0.5 wt.-% of piroctone olamine, based on the total weight of the cosmetic composition, and
from 0.02 to 7 wt.-%, preferably from 0.05 to 5 wt.-%, more preferably from 0.1 to 2.0 wt.-%, even more preferably from 0.1 to 1.0 wt.-%, particularly preferably from 0.1 to 0.5 wt.-% of the booster, based on the total weight of the cosmetic composition.

Preferably, the weight ratio of piroctone olamine to the booster is from 0.02 to 2.4, more preferably from 0.05 to 1.5, particularly preferably from 0.1 to 1.2.

More preferably, the weight ratio of piroctone olamine to the booster is from 0.02 to 0.7, more preferably from 0.05 to 0.5, particularly preferably from 0.1 to 0.4.

Also more preferably, the weight ratio of piroctone olamine to the booster is from 0.25 to 2.4, more preferably from 0.5 to 1.5, particularly preferably from 0.8 to 1.2.

Preferably, the sum of the amounts of piroctone olamine and the booster in the cosmetic composition of the invention represents at most 15 wt.-%, more preferably at most 10 wt.-%, more preferably at most 5 wt.-%, even more preferably at most 3 wt.-%, even more preferably at most 2 wt.-%, even more preferably at most 1.5 wt.-%, particularly preferably at most 1.0 wt.-%, based on the total weight of the cosmetic composition.

For example, the cosmetic composition of the invention may be a cosmetic composition selected from the group consisting of shampoo, hair conditioner, hair tonic, cream rinse, body wash, bubble bath, bath oil, facial cleanser, cleansing mask, cleansing milk, micellar water, make-up remover, cleansing wipes, perfume, soaps, shaving soaps, shaving foams, cleansing foams, face mask and body lotion.

Preferably, the cosmetic composition of the invention is a hair care composition, scalp care composition or skin care composition. More preferably, the cosmetic composition of the invention is a hair care or scalp care composition, particularly preferably a hair care and scalp care composition. Also more preferably, the cosmetic composition of the invention is a skin care composition.

Preferably, the cosmetic composition of the invention is a shampoo composition or hair conditioner composition. More preferably, the cosmetic composition of the invention is a shampoo composition, particularly preferably an anti-dandruff shampoo composition. Also more preferably, the cosmetic composition of the invention is a hair conditioner composition.

In preferred embodiments, the cosmetic composition of the invention is a shampoo composition. The shampoo composition can be in the form of rinse-off products or 'dry shampoo' products, can be opaque or transparent, and can be formulated in a wide variety of product forms, including creams, gels, emulsions, mousses and sprays. Preferably, the shampoo composition is in the form of a rinse-off product. The shampoo composition can, for example, be used on human hair and/or scalp or animal hair, preferably human hair and/or scalp.

In preferred embodiments, the cosmetic composition of the invention is a hair conditioner composition. The hair conditioner composition can be in the form of rinse-off products or leave-on products, can be opaque or transparent, and can be formulated in a wide variety of product forms, including creams, gels, emulsions, mousses and sprays. Preferably, the hair conditioner composition is in the form of a rinse-off product.

The cosmetic composition of the invention preferably comprises one or more further components (F), which can be in an amount of at least 0.01% by weight, preferably at least 0.05% by weight, more preferably at least 0.1% by weight, even more preferably at least 0.5% by weight, such as 0.5 to 50% by weight, preferably 0.5 to 35% by weight, more preferably 0.5 to 25% by weight, even more preferably 0.5 to 20% by weight of the cosmetic composition.

Preferably, the component (F) is selected from the group consisting of acidity regulators, colorants, conditioning agents, emulsifiers, film formers, fragrances, glossers, humectants, lubricants, moisturizers, pigments, preservatives, skin penetration enhancers, stabilizers, surfactants, thickeners, and viscosity modifiers. More preferably, the component (F) is selected from the group consisting of acidity regulators, glossers, lubricants, and surfactants.

Suitable lubricants are, for example, fatty alcohol components having 6 to 18 carbon atoms.

The surfactants may, for example, be selected from non-polymeric, cationic quaternary ammonium compounds, in particular cetrimonium chloride (CTAC).

Suitable classical cationic conditioning agents include cationic quaternary ammonium salts. In at least one embodiment, the component (F) is a cationic quaternary ammonium salt. Examples of such quaternary ammonium salts include benzyl triethyl ammonium chloride, cetyl trimethylammonium chloride (cetrimonium chloride, CTAC), behentrimonium chloride (BTAC) or cetylpyridinium chloride.

As cationic components, a variety of cationic polymers are suitable, including quaternized cellulose ethers, copolymers of vinylpyrrolidone, acrylic polymers, including homopolymers or copolymers of dimethyldiallylammonium chloride or acrylamide. Also suitable are various types of homo- or copolymers derived from acrylic or methacrylic acid, acrylamide, methylacrylamide, diacetone-acrylamide.

In at least one embodiment, the component (F) is a glosser. Typical glossers are silicones. Suitable as silicones are volatile or nonvolatile non-ionic silicone fluids, silicone resins, and silicone semisolids or solids. Volatile silicones are linear or cyclic silicones having a measureable vapor pressure, which is defined as a vapor pressure of at least 2 mm of mercury at 20°C. Also suitable are water insoluble nonvolatile silicone fluids including polyalkyl siloxanes, polyaryl siloxanes, polyalkylaryl siloxanes, polyether siloxane copolymers, amine-functional silicones, or mixtures thereof.

The cosmetic composition of the invention may contain from 0.05 to 5%, preferably 0.5 to 5% by weight of at least one oil component. Typical oils are organic oils, which often are esters. The oil component may comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil.

The cosmetic composition of the invention may contain from 0.05 to 5%, preferably 0.5 to 5% by weight of at least one emulsifier. Preferred emulsifiers are, for example, sorbitan esters.

The cosmetic composition of the invention can contain from 0.1 to 10% by weight, preferably from 0.2 to 5% by weight, more preferably from 0.2 to 3% by weight, also more preferably from 0.5 to 5% by weight of at least one rheology modifying agent, in particular a gelling and thickening agent. Examples are cellulosic thickeners, for example, hydroxyethylcellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulphonic acid. Other rheology modifying agents include fatty acid amides such as coconut diethanolamide and monoethanolamide, and oxyethylenated monoethanolamide of carboxylic acid alkyl ether.

Rheology modifying agents are also known as structuring materials. Common structuring materials include polymeric materials known as "carbomers", including, for example the cross-linked polyacrylic acid polymers available from Lubrizol Corporation under the trademark Carbopol^{®}. Another class of (meth)acylic acid polymers are alkali-swellable emulsion (ASE) polymers. ASE polymers include, for example, Aculyn^{®} 38 copolymer from Dow. Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration thereof is increased. In use, hydrodynamic polymers commonly give rise to compositions that exhibit shear thinning or non-Newtonian behavior. Another class of (meth)acrylic acid based rheology modifiers are hydrophobically modified alkali swellable (HASE) polymers. Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn^{®} 22 and Aculyn^{®} 28 copolymers from Dow and Aqua SF 1^{®} copolymer from Lubrizol Corporation are among the commonly used HASE materials. U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers are formed from the copolymerization of a monomer system that includes: (1) methacrylic or acrylic acid, (2) methacrylic or acrylic acid ester of a C8-C30 alkyl or, as therein more particularly described, a hydrocarbyl monoether of polyethylene glycol, (3) a C1-C4 alkyl acrylate or methacrylate, and, optionally, (4) a small amount of a polyethylenically unsaturated monomer.

The cosmetic composition of the invention (in particular hair conditioner composition) can also comprise as component (F) a fatty compound. The fatty compound may be included in the cosmetic composition at a level of from 0.1 to 20 % by weight, preferably from 1.0 to 10 % by weight. The fatty compound is selected from the group consisting of fatty alcohols (e.g. cetyl alcohol, stearyl alcohol or cetearyl alcohol), fatty acids, fatty alcohol derivatives, fatty acid derivatives, or mixtures thereof.

It is understood that the components disclosed can in some instances fall into more than one classification, e.g., some fatty alcohol derivatives can also be classified as fatty acid derivatives. However, a given classification, is not intended to be a limitation on that particular compound but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, or mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, or mixtures thereof. Examples of fatty alcohol derivatives and fatty acid derivatives include methyl stearyl ether, polyoxyethylene ethers of behenyl alcohol, ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, or mixtures thereof.

The cosmetic composition of the invention may comprise an aqueous carrier. The level and species of the aqueous carrier are selected according to the compatibility with other components and other desired characteristic of the cosmetic composition. The aqueous carrier may, for example, be water or water solutions of lower alkyl alcohols or polyhydric alcohols. The lower alkyl alcohols may, for example, be monohydric alcohols having 1 to 6 carbons, often ethanol and/or isopropanol. The polyhydric alcohols may, for example, be propylene glycol, hexylene glycol, glycerin, and/or propane diol. Preferably, the aqueous carrier is substantially water. Deionized water is preferably used. Water from natural sources, including minerals can also be used, depending on the desired characteristic of the composition. Generally, the cosmetic composition of the invention can comprise up to 80 %, often even up to 95 % by weight of water.

The cosmetic composition of the invention may also include as a further component (F) other components being suitable for rendering the compositions more cosmetically or aesthetically acceptable or to provide them with additional usage benefits. Such other components can generally be used individually at levels of from 0.001 % to 5 % by weight. A wide variety of further components (F) can be formulated into the cosmetic composition of the invention. These include conditioning agents, such as panthenol, panthenyl ethyl ether, proteins, hydrolysed proteins (preferably of vegetable or animal origin, for example hydrolysed collagen or hydrolysed keratin), nutrients; antioxidants, such as vitamin E; emollients, such as PPG-3 myristyl ether, trimethyl pentanol hydroxyethyl ether; hair-fixative polymers, such as amphoteric fixative polymers, cationic fixative polymers, anionic fixative polymers, non-ionic fixative polymers, silicone grafted copolymers; preservatives, such as benzyl alcohol, methyl paraben, propyl paraben, imidazolidinyl urea; pH adjusting agents, such as citric acid, sodium citrate, succinic acid, phosphoric acid, sodium hydroxide, sodium carbonate; salts, in general, such as potassium acetate or sodium chloride; coloring agents; hair oxidizing (bleaching) agents, such as hydrogen peroxide, perborate or persulfate salts; hair reducing agents such as thioglycolates; perfumes; and sequestering agents, such as disodium ethylene-diamine tetraacetate.

Preferably, salt is present at levels from 0.1 to 1 wt.-% of the total cosmetic composition to adjust the product viscosity. Preferably, NaOH is present at levels from 0.1 to 1 wt.-% of the total cosmetic composition to adjust the pH of the formulation.

The cosmetic composition of the invention may contain as a further component (F) a polysorbate for adjusting rheology, for example, polysorbate-20, polysorbate-21, polysorbate-40, polysorbate-60, or mixtures thereof. The polysorbate can be contained in the cosmetic composition in amounts up to 5% (e.g. 0.1 to 5%) by weight.

The cosmetic composition of the invention can also contain as a further component (F) a polypropylene glycol. Preferred polypropylene glycols are those having a weight average molecular weight of from 200 to 100000 g/mol. The polypropylene glycol may be either water-soluble, water-insoluble, or may have a limited solubility in water, depending upon the degree of polymerization and whether other moieties are attached thereto. The desired solubility of the polypropylene glycol in water will depend in large part upon the form of the composition (e.g., leave-on composition, rinse-off composition). The polypropylene glycol can be included in the cosmetic composition of the invention at a level of up to 10% by weight.

For example, in a rinse-off composition, it is preferred that the polypropylene glycol has a solubility in water at 25°C of less than about 1 g/100 g water, more preferably a solubility in water of less than about 0.5 g/100 g water, and even more preferably a solubility in water of less than about 0.1 g/100 g water. The polypropylene glycol can be included in the cosmetic composition of the invention at a level of up to 10% by weight.

The cosmetic composition of the invention can also contain, as a further component (F), low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trimethylol ester oils, citrate ester oils, or glyceryl ester oils; poly [alpha]-olefin oils; and mixtures thereof. Preferred low melting point oils are selected from the group consisting of ester oils such as pentaerythritol ester oils, trimethylol ester oils, citrate ester oils, or glyceryl ester oils; poly [alpha]-olefin oils; and mixtures thereof. Particularly useful pentaerythritol ester oils and trimethylol ester oils are pentaerythritol tetraisostearate, pentaerythritol tetraoleate, trimethylolpropane triisostearate, trimethylolpropane trioleate, or mixtures thereof. Particularly useful glyceryl esters are triisostearin, triolein or trilinolein.

The cosmetic composition of the invention can also contain, as a further component (F), a cationic polymer. Cationic polymers may be present in the cosmetic composition of the invention for further enhancing deposition performance.

Suitable cationic polymers may be homopolymers which are cationically substituted or may be formed from two or more types of monomers. The weight average (Mw) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain non-cationic spacer monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol. The cationic amines can be primary, secondary or tertiary amines, depending upon the particular species and the pH of the composition. In general, secondary and tertiary amines, especially tertiary, are preferred. Amine substituted vinyl monomers and amines can be polymerized in the amine form and then converted to ammonium by quaternization. The cationic polymers can comprise mixtures of monomer units derived from amine- and/or quaternary ammonium-substituted monomer and/or compatible spacer monomers.

Suitable cationic polymers include, for example cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; mineral acid salts of amino-alkyl esters of homo- and co-polymers of unsaturated carboxylic acids having from 3 to 5 carbon atoms, (as described in US4009256A1 from NAT STARCH CHEM CORP); cationic polyacrylamides (as described in WO95/22311A1 Unilever PLC).

Other cationic polymers that can be used include cationic polysaccharide polymers, such as cationic cellulose derivatives, cationic starch derivatives, and cationic guar gum derivatives.

Cationic polysaccharide polymers suitable for use in the cosmetic composition of the invention include monomers of the formula: A-O-[R-N⁺(R1)(R2)(R3)X⁻], wherein: A is an anhydroglucose residual group, such as a starch or cellulose anhydroglucose residual. R is an alkylene, oxyalkylene, polyoxyalkylene, or hydroxyalkylene group, or combination thereof. R1, R2 and R3 independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R1, R2 and R3) is preferably about 20 or less, and X⁻ is an anionic counterion. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the trade name Polymer LM-200. Other suitable cationic polysaccharide polymers include quaternary nitrogen-containing cellulose ethers (e.g. as described in US3962418 from L'Oréal), and copolymers of etherified cellulose and starch (e.g. as described in US3958581 from L'Oréal).

A particularly suitable type of cationic polysaccharide polymer that can be used is a cationic guar gum derivative, such as guar hydroxypropyltrimethylammonium chloride (commercially available from Solvay in their JAGUAR trade named series). Examples of such materials are JAGUAR C13S, JAGUAR C14, JAGUAR C15, JAGUAR C17, JAGUAR C16, JAGUAR CHT and JAGUAR C162.

Mixtures of any of the above cationic polymers may be used. Cationic polymer may be present in the cosmetic composition of the invention at levels of from 0.01 to 5 wt.-%, preferably from 0.05 to 1 wt.-%, more preferably from 0.08 to 0.5 wt.-% by total weight of cationic polymer based on the total weight of the cosmetic composition.

In at least one embodiment, the cationic polymers have a number average molecular weight of at least about 5000 g/mol, typically from 10000 g/mol to 10 million g/mol and are selected from the group consisting of copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Preferred cationic polymers are cationic celluloses, cationic starches, and cationic guar gums. Commercially available cationic guar polymers are e.g. Jaguar^{®} from Solvay.

In at least one embodiment, the cosmetic composition of the invention comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants. In at least one embodiment, the cosmetic composition of the invention comprises a total amount of surfactant of from 0.01 wt.-% to 70 wt.-%, from 0.1 wt.-% to 40%, from 1 wt.-% to 30%, from 2 wt.-% to 20 wt.-%.

In at least one embodiment, the cosmetic composition of the invention comprises an anionic surfactant. In at least one embodiment, the anionic surfactant is selected from the group consisting of (C10-C20)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono-, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof. Typical anionic surfactants for use in the cosmetic composition of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecyl benzene sulphonate, triethanolamine dodecylbenzene sulphonate, sodium cocoyl isethionate, sodium lauryl isethionate, lauryl ether carboxylic acid and sodium N-lauryl sarcosinate. Preferred anionic surfactants are selected from sodium lauryl sulphate and sodium lauryl ether sulphate(n)EO, where n is from 1 to 3; more preferably sodium lauryl ether sulphate(n)EO, where n is from 1 to 3; most preferably sodium lauryl ether sulphate(n)EO, where n=1. Preferably, the level of alkyl ether sulphate is from 0.5 wt.-% to 25 wt.-% of the total composition, more preferably from 3 wt.-% to 18 wt.-%, most preferably from 6 wt.-% to 15 wt.-% of the total composition.

The total amount of anionic surfactant in the cosmetic composition of the invention may range from 0.5 wt.-% to 45 wt.-%, more preferably from 1.5 wt.-% to 20 wt.-%.

The cosmetic composition of the invention may comprise fatty acyl isethionate, if present, preferably at a level of from 1 to 10 wt.-%, more preferably from 2 to 8 wt.-%, most preferably from 2.5 to 7.5 wt.-%. A preferred fatty acyl isethionate product comprises fatty acyl isethionate surfactant at a level of from 40 to 80 wt.-% of the product, as well as free fatty acid and/or fatty acid salt at a level of from 15 to 50 wt.-%. Preferably, greater than 20 wt.-% and less than 45 wt.-%, more preferably greater than 25 wt.-% and less than 45 wt.-% of the fatty acyl isethionate are of chain length greater than or equal to C16; and greater than 50 wt.-%, preferably greater than 60 wt.-% of the free fatty acid/soap is of chain length C16 to C20. In addition, the product may contain isethionate salts, which are present typically at levels less than 5 wt.-%, and traces (less than 2 wt.-%) of other impurities.

Preferably, a mixture of aliphatic fatty acids is used for the preparation of commercial fatty acyl isethionate surfactants. The resulting fatty acyl isethionate surfactants (e.g., resulting from the reaction of alkali metal isethionate and aliphatic fatty acid) preferably should have more than 20 wt.-%, preferably more than 25 wt.-%, but no more than 45 wt.-%, preferably 35 wt.-% (on basis of fatty acyl isethionate reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

In at least one embodiment, the cosmetic composition of the invention comprises an acylglycinate surfactant. In at least one embodiment, the acylglycinate surfactant conforms to the formula (Y): 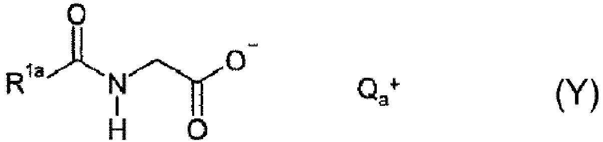 wherein
R^{1a} is a linear or branched, saturated alkyl group having 6 to 30, preferably 8 to 22, particularly preferably 8 to 18 carbon atoms or is a linear or branched, mono- or polyunsaturated alkenyl group having 6 to 30, preferably 8 to 22, particularly preferably 12 to 18 carbon atoms, and
Qₐ⁺ is a cation.

In at least one embodiment, Qₐ⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C12 alkyl or C14 alkyl. In at least one embodiment, the acylglycinate surfactant is selected from those conforming to formula (Y), wherein R is C16 alkyl or C18 alkyl.

In at least one embodiment, the cosmetic composition comprises from 0.01 wt.-% to 30 wt.-%, or from 1 wt.-% to 25 wt.-%, preferably from 5 wt.-% to 20 wt.-%, more preferably from 12 wt.-% to 18 wt.-% anionic surfactant.

In at least one embodiment, the cosmetic composition of the invention comprises a glutamate surfactant corresponding to formula (Z) or a salt thereof: 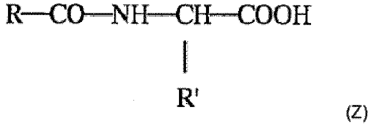
wherein R' is HOOC-CH2-CH2- or M⁺⁻OOC-CH2-CH2- wherein M⁺ is a cation; and
wherein R is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the glutamate surfactant is selected from sodium cocoyl glutamate and potassium cocoyl glutamate. In at least one embodiment, the glutamate surfactant is selected from those conforming to formula (Z), wherein R is C12 alkyl or C14 alkyl. In at least one embodiment, the glutamate surfactant is selected from those conforming to formula (Z), wherein R is C16 alkyl or C18 alkyl.

In at least one embodiment, the cosmetic composition of the invention comprises a non-ionic surfactant. Non-ionic surfactants may be present in the range 0 to 5 wt.-%. Non-ionic surfactants that can be included in the cosmetic composition of the invention include condensation products of aliphatic primary or secondary linear or branched chain alcohols or phenols with alkylene oxides, usually ethylene oxide and generally having from 6 to 30 ethylene oxide groups. Alkyl ethoxylates are particularly preferred. Most preferred are alky ethoxylates having the formula

R-(OCH2CH2)nOH,

where R is an alkyl chain of C12 to C15, and n is 5 to 9. Other suitable non-ionic surfactants include mono- or di-alkyl alkanolamides. Examples include coco mono- or di-ethanolamide and coco mono-isopropanolamide.

Further non-ionic surfactants which can be included in the cosmetic composition of the invention are alkyl polyglycosides (APGs). Typically, APG is one which comprises an alkyl group connected (optionally via a bridging group) to a block of one or more glycosyl groups. Preferred APGs are defined by the following formula:

RO-(G)n

wherein R is a branched or straight chain alkyl group which may be saturated or unsaturated, and G is a saccharide group. R may represent a mean alkyl chain length of from about C5 to about C20. Preferably R represents a mean alkyl chain length of from about C9 to about C12. G may be selected from C5 or C6 monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more. Most preferably the value of n lies between about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived non-ionic surfactants which can be included in the cosmetic composition of the invention include fatty (e.g. C10-C18) N-alkyl (C1-C6) polyhydroxy fatty acid amides, such as C12-C18 N-methyl glucamides, as described for example in WO9206154 and US5194639, and N-alkoxy polyhydroxy fatty acid amides.

In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain comprising from 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of 2 to 30 mol of ethylene oxide and/or 1 to 5 mol of propylene oxide onto C8- to C22-fatty alcohols, onto C12- to C22-fatty acids or onto alkyl phenols having 8 to 15 carbon atoms in the alkyl group, C12- to C22-fatty acid mono- and diesters of addition products of 1 to 30 mol of ethylene oxide onto glycerol, addition products of 5 to 60 mol of ethylene oxide onto castor oil or onto hydrogenated castor oil, fatty acid sugar esters, in particular esters of sucrose and one or two C8- to C22-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C8- to C22-fatty acids and a degree of ethoxylation of from 4 to 20, polyglyceryl fatty acid esters, in particular of one, two or more C8- to C22-fatty acids and polyglycerol having preferably 2 to 20 glyceryl units, alkyl glucosides, alkyl oligoglucosides and alkyl polyglucosides having C8 to C22-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminoplyethylene glycols), fatty acid ethoxylates (acylpolyethylene glycols), polypropylene glycol ethoxylates (Pluronics^{®}), fatty acid alkylol amides, (fatty acid amide polyethylene glycols), N-alkyl-, N-alkoxypoly-hydroxy-fatty acid amide, sucrose esters, sorbitol esters, polyglycol ethers, and mixtures thereof.

In at least one embodiment, the cosmetic composition of the invention comprises a fatty N-methyl-N-glucamide surfactant. In at least one embodiment, the fatty N-methyl-N-glucamide surfactant conforms to the formula (X): 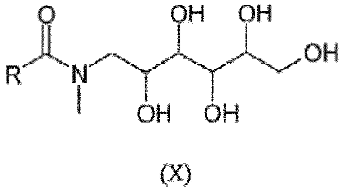 wherein
R is a linear or branched alkyl or alkenyl group having from 3 to 30 carbon atoms. In at least one embodiment, R is an alkyl group having from 3 to 30 carbon atoms. In at least one embodiment, R is a saturated aliphatic hydrocarbon group which can be linear or branched and can have from 3 to 20 carbon atoms in the hydrocarbon chain, preferably linear or branched. Branched means that a lower alkyl group such as methyl, ethyl or propyl is present as substituent on a linear alkyl chain. In at least one embodiment, R is selected from the group consisting of 1-propyl, 2-propyl, 1 butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2 methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3 methyl-2-pentyl, 4-methyl-2-pentyl, 2 methyl-3-pentyl, 3-methyl-3-pentyl, 2,2 dimethyl-1-butyl, 2,3-dimethyl-1-butyl, 3,3 dimethyl-1-butyl, 2-ethyl-1-butyl, 2,3-dimethyl-2-butyl, 3,3-dimethyl-2-butyl, 1 heptyl, 1-octyl, 1-nonyl, 1-decyl, 1 undecyl, 1-dodecyl, 1-tetradecyl, 1-hexadecyl and 1-octadecyl. Suitable fatty N methyl-N-glucamide surfactants are described in WO2013/178700 and EP 0 550 637, which are incorporated herein by reference. In at least one embodiment, the N-methyl-N-glucamide surfactant is selected from those conforming to formula (X), wherein R is C12 alkyl or C14 alkyl. In at least one embodiment, the N-methyl-N-glucamide surfactant is selected from those conforming to formula (X), wherein R is C16 alkyl or C18 alkyl.

In at least one embodiment, the cosmetic composition of the invention comprises from 1 wt.-% to 20 wt.-%, more preferably from 2 wt.-% to 10 wt.-%, even more preferably from 3 wt.-% to 7 wt.-% non-ionic surfactant.

Amphoteric or zwitterionic surfactant can be included in the cosmetic composition of the invention in an amount ranging from 0.5 wt.-% to about 8 wt.-%, preferably from 1 wt.-% to 4 wt.-% of the total composition.

In at least one embodiment, the amphoteric surfactants are selected from the group consisting of N-(C₁₂-C₁₈)-alkyl-beta-aminopropionates and N-(C₁₂-C₁₈)-alkyl-beta-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine; (C₁₂-C₁₈)-alkyl-dimethyl-sulfopropylbetaine; amphosurfactants based on imidazoline (trade name: Miranol^{®}, Steinapon^{®}), preferably the sodium salt of 1-(beta-carboxymethyloxyethyl)-1-(carboxymethyl)-2-laurylimidazolinium; amine oxide, e.g., (C₁₂-C₁₈)-alkyl-dimethylamine oxide, fatty acid amidoalkyldimethylamine oxide, and mixtures thereof.

In at least one embodiment, the cosmetic composition of the invention comprises a betaine surfactant. Optionally, the betaine surfactant is selected from C8- to C18-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. Optionally, the betaine surfactant is selected from C8- to C18-sulfobetaines. In at least one embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. Optionally, the betaine surfactant is selected from carboxyl derivatives of imidazole, the C8- to C18-alkyldimethylammonium acetates, the C8- to C18 alkyldimethylcarbonylmethylammonium salts, and the C8- to C18-fatty acid alkylamidobetaines, and mixtures thereof. Optionally, the C8- to C18-fatty acid alkylamidobetaine is selected from coconut fatty acid amidopropylbetaine, N-coconut fatty acid amidoethyl-N-[2-(carboxymethoxy)ethyl]glycerol (CTFA name: cocoamphocarboxyglycinate), and mixtures thereof. A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In at least one embodiment, the cosmetic composition of the invention comprises from 0.5 wt.-% to 20 wt.-%, preferably from 1 wt.-% to 10 wt.-% amphoteric surfactant.

In at least one embodiment, the cosmetic composition of the invention comprises a surfactant system. In at least one embodiment, the surfactant system comprises at least one surfactant selected from the group consisting of lauryl sulfate, laureth sulfate, cocamidopropyl betaine, sodium cocoylglutamate, lauroamphoacetate, and mixtures thereof. In at least one embodiment, the surfactant system comprises sodium laureth sulfate, sodium lauryl sulfate, and optionally cocamidopropyl betaine. In at least one embodiment, the surfactant system comprises sodium laureth sulfate, potassium cocoylglutamate, and cocamidopropyl betaine.

In at least one embodiment, the cosmetic composition of the invention contains as a further component a silicone compound. The cosmetic composition can comprise up to 5% (e.g. 0.1 to 5%) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. Silicone compounds can be available as silicone oils or emulsions. The silicone compounds may further be incorporated in the present composition in the form of an emulsion, wherein the emulsion is pre-made and added to the formulation, or made during the formulation process by mechanical mixing with or without the aid of an additional surfactant selected from anionic surfactants, non-ionic surfactants, cationic surfactants, and mixtures thereof.

In at least one embodiment, the cosmetic composition of the invention contains silicone conditioning agents. Preferably, these are emulsified droplets of a silicone conditioning agent. These are for enhancing conditioning performance.

Suitable silicones include polydiorganosiloxanes, in particular polydimethylsiloxanes, which have the CTFA designation dimethicone. Also suitable for use in the cosmetic composition of the invention are polydimethyl siloxanes having hydroxyl end groups, which have the CTFA designation dimethiconol. Also suitable for use in the cosmetic composition of the invention are silicone gums having a slight degree of cross-linking, as are described for example in WO 96/31188. The viscosity of the emulsified silicone itself (not the emulsion or the final composition) is typically at least 10,000 cSt at 25°C. The viscosity of the silicone itself is preferably at least 60,000 cSt, most preferably at least 500,000 cSt, ideally at least 1,000,000 cSt. Preferably, the viscosity does not exceed 1x10⁹ cSt for ease of formulation. Emulsified silicones for use in the cosmetic composition of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation. A further preferred class of silicones for inclusion in the cosmetic composition of the invention are amino functional silicones. By "amino functional silicone" is meant a silicone containing at least one primary, secondary or tertiary amine group, or a quaternary ammonium group. Examples of suitable amino functional silicones include polysiloxanes having the CTFA designation "amodimethicone". Specific examples of amino functional silicones suitable for use in the cosmetic composition of the invention are the aminosilicone oils DC2-8220, DC2-8166 and DC2-8566 (all ex Dow Corning). Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactants. Pre-formed emulsions of amino functional silicones are also available from suppliers of silicone oils such as Dow Corning and General Electric. Specific examples include DC939 Cationic Emulsion and the non-ionic emulsions DC2-7224, DC2- 8467, DC2-8177 and DC2-8154 (all ex Dow Corning).

Combination of amino and non-amino functional silicones may also be used.

The total amount of silicone is preferably from 0.01 wt.-% to 10 wt.-% of the total composition, more preferably from 0.1 wt.-% to 5 wt.-%, most preferably from 0.5 wt.-% to 3 wt.-%.

In at least one embodiment, the cosmetic composition of the invention comprises a preservative or preservative system. Examples of suitable preservatives include benzyl alcohol, phenoxyethanol, parabens, benzoic acid/sodium benzoate, sorbic acid/potassium sorbate, and other organic acids used to provide antimicrobial protection. In at least one embodiment, the cosmetic composition comprises from 0.01 to 5 wt.-%, particularly preferably from 0.05 to 1 wt.-% of at least one preservative. Suitable preservatives are the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the cosmetic composition comprises a preservative selected from the group consisting of cetyltrimethyl ammonium chloride, cetylpyridinium chloride, benzethonium chloride, diisobutylethoxyethyldimethyl benzylammonium chloride, sodium N-lauryl sarcosinate, sodium-N-palmethylsarcosinate, lauroylsarcosine, N-myristoylglycine, potassium-N-laurylsarcosine, trimethylammonium chloride, sodium aluminium chlorohydroxylactate, triethylcitrate, tricetylmethylammonium chloride, 2,4,4'-trichloro-2'-hydroxydiphenylether (Triclosan), phenoxyethanol, 3,4,4'-trichlorocarbanilide (Triclocarban), diaminoalkylamide, L-lysine hexadecylamide, heavy metal citrate salts, zinc salts, zinc phenol sulfate, farnesol, naftifine, terbinafine, selenium disulfide, methylchloroisothiazolinone, methylisothiazolinone, methyldibromo glutaronitrile, AgCI, chloroxylenol, sodium salts of diethylhexylsulfosuccinate, sodiumbenzoate, phenoxyethanol, benzyl alcohol, phenoxyisopropanol, paraben, such as butyl-, ethyl-, methyl- und propylparaben, and their salts, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the cosmetic composition of the invention is substantially free of parabens.

The cosmetic composition of the invention may also comprise a dispersed, nonvolatile, water-insoluble oily conditioning agent. By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols. Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used. Particularly preferred fatty esters are mono-, di- and triglycerides, more specifically the mono-, di-, and tri-esters of glycerol and long chain carboxylic acids such as C8-C22 carboxylic acids. Preferred materials include cocoa butter, palm stearin, sunflower oil, soybean oil and coconut oil.

The oily or fatty material may be present at a level of from 0.05 to 10 wt.-%, preferably from 0.2 to 5 wt.-%, more preferably from 0.5 to 3 wt.-%, based on the total weight of the cosmetic composition.

In a particularly preferred embodiment, the composition of the invention is a shampoo composition.

In at least one embodiment, the shampoo composition comprises from 1 to 99%, preferably from 5 to 95%, more preferably from 10 to 90% by weight of the total composition of water, and from 0.1 to 99%, preferably from 1 to 95%, more preferably from 5 to 90%, often from 5 to 25% by weight of the total composition of a cleansing surfactant. Suitable cleansing surfactants are generally anionic, amphoteric, betaine, or zwitterionic surfactants. For example, the anionic surfactants are alkyl ether or alkyl ether sulfates, such as sodium lauryl sulfate, or other compounds described above.

In at least one embodiment, the shampoo composition comprises one or more further cosmetically acceptable components (F), which can be present in an amount of at least 0.5% by weight, or from 0.5 to 20% by weight, by total weight of the shampoo composition. Preferably, the component (F) is selected from the group consisting of cleansing ingredients, acidity regulators, colorants, conditioning agents, emulsifiers, film formers, fragrances, glossers, humectants, lubricants, moisturizers, pigments, preservatives, hair penetration enhancers, scalp actives, stabilizers, surfactants, thickeners, viscosity modifiers, and combinations thereof. More preferably, the component (F) is selected from the group consisting of surfactants, viscosity-modifying polymers and conditioning ingredients. In at least one embodiment, the shampoo composition comprises from 0.05 wt.-% to 5 wt.-% (preferably from 0.1 wt.-% to 2.0 wt.-%, more preferably from 0.1 wt.-% to 1.0 wt.-%, particularly preferably from 0.1 wt.-% to 0.5 wt.-%) of piroctone olamine, from 0.05 wt.-% to 5 wt.-% (preferably from 0.1 wt.-% to 2.0 wt.-%, more preferably from 0.1 wt.-% to 1.0 wt.-%, particularly preferably from 0.1 wt.-% to 0.5 wt.-%) of the booster described herein, and at least 0.5% by weight of one or more further components (F) selected from the group consisting of surfactants, polymers, conditioning agents, actives, acidity regulators, lubricants, moisturizers, oils, preservatives, sequestrants, strengtheners, sun protectors, and combinations thereof.

In at least one embodiment, the shampoo composition comprises further cosmetically acceptable components (F) being cleansing ingredients. In at least one embodiment, the shampoo composition comprises from 0.05 to 20% by weight of cleansing ingredients, based on the total weight of the shampoo composition. In at least one embodiment of the shampoo composition, the level of cleansing ingredient is from 1% to 20% by weight, preferably from 5% to 18%, more preferably from 8% to 16%, based on the total weight of the shampoo composition. In at least one embodiment, the cleansing ingredient is selected from the group consisting of non-polymeric surfactants, saponins, polymeric surfactants, and combinations thereof. Preferably, the cleansing ingredient comprises or consists of surfactants.

In at least one embodiment, the shampoo composition comprises from 0.05 wt.-% to 5 wt.-% (preferably from 0.1 wt.-% to 2.0 wt.-%, more preferably from 0.1 wt.-% to 1.0 wt.-%, particularly preferably from 0.1 wt.-% to 0.5 wt.-%) of piroctone olamine, from 0.05 wt.-% to 5 wt.-% (preferably from 0.1 wt.-% to 2.0 wt.-%, more preferably from 0.1 wt.-% to 1.0 wt.-%, particularly preferably from 0.1 wt.-% to 0.5 wt.-%) of the booster described herein, and at least 0.5 % by weight of surfactants, preferably cleansing anionic or non-ionic surfactants, such as sodium laureth sulphate, sodium lauryl sulphate, ammonium laureth sulphate, ammonium lauryl sulphate, olefin sulfonates, olefin sulfates, laureth-3 or 4, cocamide DEA, glucosides, cocamidopropyl betaine, coco betaine, cocoamphodipropionate, sodium methyl 2-sulfolaurate and other laurates, sulfoacetates, sulfosuccinates, lactylates, sultaines, caprylates/ caprates, isethionates, glutamates, taurates, sarcosinates, glucamides, and combinations thereof.

In at least one embodiment, the shampoo composition is silicone-free. In at least one embodiment, the shampoo composition is sulfate-free. In at least one embodiment, the shampoo composition is silicone-free and sulfate-free.

In at least one embodiment, the shampoo composition comprises, based on the total weight of the shampoo composition:
(i) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of piroctone olamine;
(ii) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of the booster described herein;
(iii) from 5 wt.-% to 20 wt.-% of one or more anionic surfactants;
(iv) at least 50 wt.-% water; and
(v) at least one further cosmetically acceptable component (F) selected from the group consisting of silicone, cationic polymer, rheology modifiying agent, and an amphoteric or zwitterionic surfactant.

In at least one embodiment, the shampoo composition comprises, based on the total weight of the shampoo composition:
(i) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of piroctone olamine;
(ii) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of the booster described herein;
(iii) from 5 wt.-% to 20 wt.-% of one or more anionic surfactants;
(iv) at least 50 wt.-% water;
(v) at least one further component selected from the group consisting of silicone, cationic polymer, rheology modifiying agent, and an amphoteric or zwitterionic surfactant; and
(v) at least one further cosmetically acceptable component (F).

In at least one embodiment, the shampoo composition consists of, based on the total weight of the shampoo composition:
(i) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of piroctone olamine;
(ii) from 0.05 wt.-% to 5 wt.-%, preferably from 0.1 wt.-% to 2.0 wt.-%, more preferably from 0.1 wt.-% to 1.0 wt.-%, particularly preferably from 0.1 wt.-% to 0.5 wt.-% of the booster described herein;
(iii) from 5 wt.-% to 20 wt.-% of one or more anionic surfactants;
(iv) at least 50 wt.-% water;
(v) at least one further component selected from the group consisting of silicone, cationic polymer, rheology modifiying agent, and an amphoteric or zwitterionic surfactant; and
(vi) at least one further cosmetically acceptable component (F) selected from the group consisting of conditioning agents, such as panthenol, panthenyl ethyl ether, proteins, hydrolysed proteins (preferably of vegetable or animal origin, for example hydrolysed collagen or hydrolysed keratin), nutrients; antioxidants, such as vitamin E; emollients, such as PPG-3 myristyl ether, trimethyl pentanol hydroxyethyl ether; hair-fixative polymers such as amphoteric fixative polymers, cationic fixative polymers, anionic fixative polymers, non-ionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents, such as the thioglycolates; perfumes; and sequestering agents, such as disodium ethylene-diamine tetraacetate.

The cosmetic composition of the invention can be prepared by methods known in the art. For example, the cosmetic composition of the invention can be prepared by mixing its ingredients. Piroctone olamine and the booster may be added separately or in the form of a blend. In preferred embodiments, piroctone olamine and the booster are added separately. In also preferred embodiments, piroctone olamine and the booster are added in the form of a blend. In particular embodiments, piroctone olamine and the booster are added in the form of a blend of the invention. The cosmetic composition of the invention may be prepared using the blend of the invention.

The invention also relates to the use of piroctone olamine and a booster as an anti-dandruff system or as a preservative system, wherein the booster is selected from alcohols having 2 to 5 hydroxy groups and plant extracts.

In preferred embodiments, the invention relates to the use of piroctone olamine and a booster as an anti-dandruff system.

In preferred embodiments, the invention relates to the use of piroctone olamine and a booster as a preservative system.

Particularly preferably, piroctone olamine and the booster are part of a cosmetic composition. Preferably, the cosmetic composition is a shampoo composition or hair conditioner composition. More preferably, the cosmetic composition is a shampoo composition, particularly preferably an anti-dandruff shampoo composition. Also more preferably, the cosmetic composition of the invention is a hair conditioner composition.

Preferably, the dandruff is caused by dandruff-causing organisms, more preferably Malassezia species, even more preferably Malassezia furfur and/or Malassezia globosa, particularly preferably Malassezia furfur, also particularly preferably Malassezia globosa.

One or more boosters may be used. Preferably, 1 to 3 boosters are used, more preferably 1 or 2 boosters, particularly preferably 1 booster, also particularly preferably 2 boosters.

In preferred embodiments, the booster is selected from alcohols having 2 to 5 hydroxy groups. Preferred alcohols having 2 to 5 hydroxy groups are described further above.

In preferred embodiments, the booster is selected from plant extracts. Preferred plant extracts are described further above.

In particular embodiments, a booster selected from alcohols having 2 to 5 hydroxy groups and a booster selected from plant extracts are used.

Preferably, the weight ratio of piroctone olamine to the booster is from 0.02 to 2.4, more preferably from 0.05 to 1.5, particularly preferably from 0.1 to 1.2.

More preferably, the weight ratio of piroctone olamine to the booster is from 0.02 to 0.7, more preferably from 0.05 to 0.5, particularly preferably from 0.1 to 0.4.

Also more preferably, the weight ratio of piroctone olamine to the booster is from 0.25 to 2.4, more preferably from 0.5 to 1.5, particularly preferably from 0.8 to 1.2.

Piroctone olamine and the booster may be used separately or in the form of a blend. In preferred embodiments, piroctone olamine and the booster are used separately. In also preferred embodiments, piroctone olamine and the booster are used in the form of a blend. In particular embodiments, piroctone olamine and the booster are used in the form of a blend of the invention.

The invention also relates to a method of treating hair and/or scalp, comprising:
a) applying a shampoo composition and/or hair conditioner composition (preferably shampoo composition) as described herein onto wet hair and/or scalp and then
b) removing the shampoo composition and/or hair conditioner composition (preferably shampoo composition) from the hair and/or scalp.

The invention also relates to the use of a booster to enhance the antimicrobial activity of piroctone olamine, wherein the booster is selected from alcohols having 2 to 5 hydroxy groups and plant extracts.

The invention also relates to a compound of formula (I), 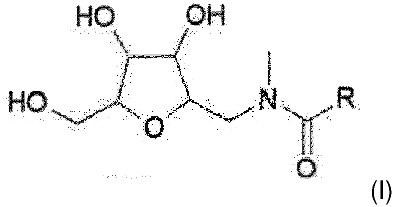 wherein R is selected from saturated hydrocarbon chains having 11 to 23 carbon atoms and unsaturated hydrocarbon chains having 5 to 23 carbon atoms. Preferably, R in formula (I) is selected from saturated hydrocarbon chains having 11 to 17 carbon atoms and unsaturated hydrocarbon chains having 7 to 17 carbon atoms. More preferably, R in formula (I) is selected from saturated hydrocarbon chains having 11 to 13 carbon atoms and unsaturated hydrocarbon chains having 7 to 13 carbon atoms.

In preferred embodiments, R in formula (I) is selected from saturated hydrocarbon chains having 11 to 23 carbon atoms, preferably 11 to 17 carbon atoms, more preferably 11 to 13 carbon atoms. Particularly preferably, R in formula (I) is - (CH₂)₁₀CH₃, -(CH₂)₁₂CH₃, or mixtures thereof.

In preferred embodiments, R in formula (I) is selected from unsaturated hydrocarbon chains having 5 to 23 carbon atoms, preferably 7 to 17 carbon atoms, more preferably 7 to 13 carbon atoms. Particularly preferably, the R-C=O residue in formula (I) is derived from 9-decenoic acid, 9-dodecenoic acid, or mixtures thereof.

Also particularly preferably, the R-C=O residue in formula (I) is derived from cocoyl fatty acids.

The compounds of formula (I) can, for example, be prepared from N-methyl-glucamine and fatty acid R-COOH. For example, the compounds of formula (I) are prepared by a process comprising the steps:
(a) contacting N-methyl-glucamine with fatty acid R-COOH to form a reaction blend;
(b) heating the reaction blend to boil off any water;
(c) allowing the reaction blend to react for at least 1 hour.

Preferably, step (b) occurs at at least 100 °C, particularly preferably from 120 °C to 150 °C. Preferably, step (b) occurs under vacuum, for example at 30 mbar. Preferably, step (c) occurs for at least 3 hours, particularly preferably at least 6 hours. Preferably, step (c) occurs at at least 100 °C, more preferably from 120 °C to 180 °C, particularly preferably from 140 °C to 170 °C. Preferably, step (c) occurs under nitrogen gas.

The invention is further illustrated by the following examples, without being limited thereby.

### Examples

### Example 1

Determination of minimal inhibitory concentration (MIC) using a multipoint inoculator

### SCOPE

The value determined is the minimal inhibitory concentration (MIC) of anti-microbial agent necessary to reduce a known bacterial/yeast/fungi population to zero after a specified contact time.

### PRINCIPLE

A known aliquot of chemical is dosed into molten agar to achieve the test concentration. This is poured into a sterile Petri dish and allowed to solidify. A multipoint inoculator is used to inoculate the surface of the agar plate. The microbial species can be bacteria, yeast or fungi dependent on the agar used or chemical under test. After the determined time interval(s), plates are analyzed for growth or no growth for the organisms under test. By using a control plate, the level at which the chemical will provide zero growth can be determined.

### REAGENTS

### Malassezia agar:

47.0 g Sabouraud 2% glucose agar (Merck 1.07315.0500)
+ 10 g Yeast extract granulated (Merck 1.03753.0500)
+ 10 g Peptone from soy (VWR N454-500G)
+ 10 g Tween 80 (Polysorbate 80) (Merck 8.17061.1000)
+ 20 ml Olive oil (Sigma-Aldrich 75343-1L)
Up to 1000 ml with demineralized water

### Malassezia agar slopes:

Prepared with Malassezia agar

### Maximum recovery diluent (VWR 1.12535.0500):

8.5 g Sodium chloride
+ 1 g Peptone
Up to 1000 ml with demineralized water

### Chemicals under test:

Piroctone olamine solution, booster solution and solution containing 1:1 mixture of piroctone olamine and booster are tested in each run.

### APPARATUS

Usual laboratory equipment but specifically a sterilized multipoint inoculator.

### ANALYTICAL PROCEDURE

### Preparation of Yeast inoculum:

- For any yeast species to be used, streak onto Malassezia Agar slopes and incubate for 48 h to obtain luxuriant growth.
- Add 9 ± 0.1 ml maximum recover diluent to the yeast test tubes and vortex to produce a homogenous suspension.

### Organisms:

| | |
|---|---|
| • Malassezia furfur | DSM 6170 |
| • Malassezia globosa | ATCC 4889 |
| • Malassezia globosa | ATCC 4794 |

### CHEMICAL TEST SOLUTIONS

• Accurately weigh out the chemical to be tested into a sterile container, e.g. glass beaker or bottle.
• Dissolve in 1,2-propandiol.
• Add the following active concentrations to liquid agar at 45°C:

| | | |
|---|---|---|
| 250 ppm | 500 ppm | 750 ppm |
| 1000 ppm | 1500 ppm | 2000 ppm |
| 3000 ppm | 4000 ppm | 6000 ppm |
| 8000 ppm | 10000 ppm | |

• Homogenize by shaking.
• Fill 20-25 ml each in 2 Petri dishes and allow to solidify.

Control plates containing no chemical are prepared at the same time as the dosed samples.

### INOCULATION OF PLATES

- Fill the relevant number of wells of the sterilized multipoint inoculator using a sterile syringe or pipette.
- Depress the needles of the inoculator into the wells, remove and transfer onto the surface of a solid agar plate. Each pass of the inoculator deposits approximately 10 µl of organism on the agar plate surface.
- Incubate the plates at a temperature of 25 ± 2.5°C for 48/72h.

### RESULTS

The results are reported as the lowest concentration to show no growth on each of the duplicate plates for each test culture. Data are based on comparisons to the control sample. The results are given in the following Tables 1a to 1f.

**Table 1a:**

| **MIC [ppm]** | Sorbitan caprylate (Velsan^{®} SC) | Piroctone olamine + sorbitan caprylate | Piroctone olamine (Octopirox^{®}) |
|---|---|---|---|
| Malassezia furfur DSM 6170 | 3000 | 2500 | 4000 |

Table 1a demonstrates a synergistic effect of piroctone olamine and sorbitan caprylate against Malassezia furfur. The combination of piroctone olamine and sorbitan caprylate shows efficient performance versus dandruff causing organisms.

**Table 1b:**

| **MIC [ppm]** | Capryloyl/caproyl anhydro methyl glucamide (Velsan^{®} Flex) | Piroctone olamine + capryloyl/caproyl anhydro methyl glucamide | Piroctone olamine (Octopirox^{®}) |
|---|---|---|---|
| Malassezia globosa ATCC 4889 | 500 | 500 | 750 |

Table 1b demonstrates a synergistic effect of piroctone olamine and capryloyl/caproyl anhydro methyl glucamide against Malassezia globosa. The combination of piroctone olamine and capryloyl/caproyl anhydro methyl glucamide shows efficient performance versus dandruff causing organisms.

**Table 1c:**

| **MIC [ppm]** | Ethylhexylglycerin (Velsan^{®} EHG) | Piroctone olamine + ethylhexylglycerin | Piroctone olamine (Octopirox^{®}) |
|---|---|---|---|
| Malassezia furfur DSM 6170 | 750 | 500 | 4000 |
| Malassezia globosa ATCC 4889 | 500 | < 250 | 750 |

Table 1c demonstrates a synergistic effect of piroctone olamine and ethylhexylglycerin against Malassezia furfur and Malassezia globosa. The combination of piroctone olamine and ethylhexylglycerin shows efficient performance versus dandruff causing organisms.

**Table 1d:**

| **MIC [ppm]** | Caprylyl glycol | Piroctone olamine + caprylyl glycol | Piroctone olamine (Octopirox^{®}) |
|---|---|---|---|
| Malassezia furfur DSM 6170 | 1500 | 1500 | 4000 |

Table 1d demonstrates a synergistic effect of piroctone olamine and caprylyl glycol against Malassezia furfur. The combination of piroctone olamine and caprylyl glycol shows efficient performance versus dandruff causing organisms.

**Table 1e:**

| **MIC [ppm]** | Caprylyl glyceryl ether (Velsan^{®} CGE) | Piroctone olamine + caprylyl glyceryl ether | Piroctone olamine (Octopirox^{®}) |
|---|---|---|---|
| Malassezia furfur DSM 6170 | 750 | 750 | 4000 |

Table 1e demonstrates a synergistic effect of piroctone olamine and caprylyl glyceryl ether against Malassezia furfur. The combination of piroctone olamine and caprylyl glyceryl ether shows efficient performance versus dandruff causing organisms.

**Table 1f:**

| **MIC [ppm]** | 1,2-Hexanediol | Piroctone olamine + 1,2-hexanediol | Piroctone olamine (Octopirox^{®}) |
|---|---|---|---|
| Malassezia globosa ATCC 4794 | 2500 | 2500 | 4000 |

Table 1f demonstrates a synergistic effect of piroctone olamine and 1,2-hexanediol against Malassezia globosa. The combination of piroctone olamine and 1,2-hexanediol shows efficient performance versus dandruff causing organisms.

### Example 2

Determination of minimal inhibitory concentration (MIC) using a microtitre plate

### SCOPE

The value determined is the minimal inhibitory concentration (MIC) of anti-microbial agent necessary to prevent the growth of a named microbial species under the conditions specified.

### PRINCIPLE

The microtitre plate method utilizes a 96 'U' shaped well plate. Each well has a capacity of 400 µl. A known biocide concentration or concentrations of biocides are pipetted into a row or column of wells. This is then double diluted using a dilute nutrient solution until all wells have been used.

A known inoculum of Malassezia species is then added, and the plate is incubated at an appropriate temperature and time for the organism under study. After the incubation period, the plates are examined and scored using the first row or column as the control (no added biocide). The orientation of the well dictates the number of biocide concentrations that can be used. There are no limitations on the starting concentration of the solutions used. By using a series of dilutions of the same solution, it is possible to give a narrow range for biocidal activity against the specified target organism.

### REAGENTS

### RPMI Optimum:

RPMI 1640 (500 ml) (VWR L0500-500)
+ 17.3 g MOPS (Merck 1370781000)
+ 10.0 g Glucose (Merck 1.08337.1000)
+ 2.5 g Glycerol (Merck 8.18709.1000)
+ 2.5 g Tween 60 (Polysorbate) (Merck 8.170762.500)
+ 20 ml Oleic acid (VWR 881231636)
+ 12.5 µg/ml Resazurin (VWR 199303-5g)

### Malassezia agar:

47.0 g Sabouraud 2% glucose agar (Merck 1.07315.0500)
+ 10 g Yeast extract granulated (Merck 1.03753.0500)
+ 10 g Peptone from soy (VWR N454-500G)
+ 10 g Tween 80 (Polysorbate 80) (Merck 8.17061.1000)
+ 20 ml Olive oil (Sigma-Aldrich 75343-1L)
Up to 1000 ml with demineralized water

### Malassezia agar slopes:

Prepared with Malassezia agar

### Maximum recovery diluent (VWR 1.12535.0500):

8.5 g Sodium chloride
+ 1 g Peptone
Up to 1000 ml with demineralized water

### Stock solutions:

Piroctone olamine stock solution(s), piroctone olamine + booster stock solutions, booster stock solutions prepared in DMSO or prepared in a suitable diluent.

### APPARATUS

Usual laboratory equipment but specifically sterile microtitre plates and lids, and a multi-channel pipette.

### ANALYTICAL PROCEDURE

### Yeast:

A loopful of culture is removed from a Malassezia agar slope stored at 35°C and homogenized in 10 ml maximum recovery diluent. This is vortexed for 30 seconds and the suspension is adjusted using maximum recovery diluent so that the solution contains approximately 1-5x10⁵ viable cells/ml. The actual number in the inoculum is determined using a standard spread plate count technique.

| | | |
|---|---|---|
| Organism: | From: | Ref number: |
| Malassezia furfur | DSMZ | 6170 |

### Preparation of microtitre plate:

- Pipette 100 µl of sterile RPMI Optimum solution into each well of a 96 well plate.
- Into the wells of the third row pipette 90 µl of sterile RPMI Optimum solution.
- Then add 10 ppm piroctone olamine solution or piroctone olamine + booster solution or booster solution under test into the wells of the third row. (The wells of row 1 and 2 act as controls. Therefore, piroctone olamine solution or piroctone olamine + booster solution or booster solution addition begins with row 3). The solution is therefore diluted 2-fold.
- Serially dilute this down from row 2-12 using 100 µl aliquots. Discard the last 100 µl. The solution has therefore been diluted 2-fold each time.
- Add 10 µl of a 1-5x10⁵ CFU's/ml inoculum to each well and mix on each addition.
- Incubate the plates at 35°C temperature for 48 hours.

### RESULTS

Cell growth is quantified using the indicator resazurin which shows a characteristic colour change from blue to pink in the presence of viable cells. MIC values are determined visually by selecting the well with the lowest ppm value which remains blue. The results are given in the following Table 2.

**Table 2:**

| **Entry** | **Piroctone olamine (Octopirox^{®})** | **Booster** | | **MIC [ppm]** |
|---|---|---|---|---|
| 2a | 100% | - | | 3.90 |
| 2b | 70% | - | | 5.46 |
| 2c | 70% | Ethylhexylglycerin (Velsan^{®} EHG) | 30% | 3.90 |
| 2d | 70% | Lauryl/myristyl anhydro methyl glucamide* | 30% | 4.68 |
| 2e | 70% | N-9-dodecenoyl-N-methylglucamine** | 30% | 4.64 |
| 2f | 70% | Caprylyl glycol | 30% | 3.90 |
| 2g | - | Ethylhexylglycerin (Velsan^{®} EHG) | 100% | > 25 ppm |
| 2h | - | Ethylhexylglycerin (Velsan^{®} EHG) | 30% | > 25 ppm |
| 2i | - | Caprylyl glycol | 100% | > 25 ppm |
| 2j | - | Caprylyl glycol | 30% | > 25 ppm |

| | | | | |
|---|---|---|---|---|
| * Compound of formula (I) wherein R is -(CH₂)₁₀CH₃ / -(CH₂)₁₂CH₃ ** Compound of formula (II) wherein the R^{a}-C=O residue is derived from 9-dodecenoic acid | | | | |

Table 2 demonstrates a synergistic effect of piroctone olamine and the respective booster (ethylhexylglycerin, lauryl/myristyl anhydro methyl glucamide, N-9-dodecenoyl-N-methylglucamine, caprylyl glycol) against Malassezia furfur. The combination of piroctone olamine and the respective booster shows the same efficient performance versus dandruff causing organisms as piroctone olamine itself.

### Examples 3 and 4

### Blends

| **Example** | **3a** | **3b** | **3c** | **3d** | **3e** | **3f** | **3g** |
|---|---|---|---|---|---|---|---|
| Ingredient | wt.-% | wt.-% | wt.-% | wt.-% | wt.-% | wt.-% | wt.-% |
| Piroctone olamine | 2 | 5 | 10 | 15 | 20 | 50 | 70 |
| Ethylhexylglycerin | 98 | 95 | 90 | 85 | 80 | 50 | 30 |
| Total | 100 | 100 | 100 | 100 | 100 | 100 | 100 |

| **Example** | **4a** | **4b** | **4c** | **4d** | **4e** | **4f** | **4g** |
|---|---|---|---|---|---|---|---|
| Ingredient | wt.-% | wt.-% | wt.-% | wt.-% | wt.-% | wt.-% | wt.-% |
| Piroctone olamine | 2 | 5 | 10 | 15 | 20 | 50 | 70 |
| Caprylyl glycol | 98 | 95 | 90 | 85 | 80 | 50 | 30 |
| Total | 100 | 100 | 100 | 100 | 100 | 100 | 100 |

The blends can be prepared by mixing their ingredients. The blends are particularly useful for anti-dandruff applications and/or for preserving cosmetic compositions.

### Examples 5 and 6

### Blends

| **Example** | **5a** | **5b** | **5c** | **5d** | **5e** | **5f** | **5g** |
|---|---|---|---|---|---|---|---|
| Ingredient | wt.-% | wt.-% | wt.-% | wt.-% | wt.-% | wt.-% | wt.-% |
| Piroctone olamine | 2 | 5 | 10 | 15 | 20 | 50 | 70 |
| Garcinia Mangostana Peel Extract | 98 | 95 | 90 | 85 | 80 | 50 | 30 |
| Total | 100 | 100 | 100 | 100 | 100 | 100 | 100 |

| **Example** | **6a** | **6b** | **6c** | **6d** | **6e** | **6f** | **6g** |
|---|---|---|---|---|---|---|---|
| Ingredient | wt.-% | wt.-% | wt.-% | wt.-% | wt.-% | wt.-% | wt.-% |
| Piroctone olamine | 2 | 5 | 10 | 15 | 20 | 50 | 70 |
| Orthosiphon Stamineus Leaf Extract | 98 | 95 | 90 | 85 | 80 | 50 | 30 |
| Total | 100 | 100 | 100 | 100 | 100 | 100 | 100 |

The blends can be prepared by mixing their ingredients. The blends are particularly useful for anti-dandruff applications and/or for preserving cosmetic compositions.

### Example 7

### Blends

| **Example** | **7a** | **7b** | **7c** | **7d** | **7e** | **7f** |
|---|---|---|---|---|---|---|
| Ingredient | wt.-% | wt.-% | wt.-% | wt.-% | wt.-% | wt.-% |
| Piroctone olamine | 15 | 15 | 15 | 15 | 15 | 15 |
| Ethylhexylglycerin | 75 | 75 | 70 | 70 | 80 | 80 |
| Glycerin | 10 | - | - | - | - | - |
| Water | - | 10 | - | - | - | - |
| Ethanol | - | - | 15 | - | - | - |
| Propylene glycol | - | - | - | 15 | - | - |
| Sodium xylenesulfonate | - | - | - | - | 5 | - |
| Sodium cumenesulfonate | - | - | - | - | - | 5 |
| Total | 100 | 100 | 100 | 100 | 100 | 100 |

| **Example** | **7g** | **7h** | **7i** | **7j** | **7k** | **7l** |
|---|---|---|---|---|---|---|
| Ingredient | wt.-% | wt.-% | wt.-% | wt.-% | wt.-% | wt.-% |
| Piroctone olamine | 45 | 45 | 45 | 45 | 45 | 45 |
| Ethylhexylglycerin | 45 | 45 | 40 | 40 | 50 | 50 |
| Glycerin | 10 | - | - | - | - | - |
| Water | - | 10 | - | - | - | - |
| Ethanol | - | | 15 | - | - | - |
| Propylene glycol | - | - | - | 15 | - | - |
| Sodium xylenesulfonate | - | - | - | - | 5 | - |
| Sodium cumenesulfonate | - | - | - | - | - | 5 |
| Total | 100 | 100 | 100 | 100 | 100 | 100 |

The blends can be prepared by mixing their ingredients. The blends are particularly useful for anti-dandruff applications and/or for preserving cosmetic compositions.

### Example 8

### Blends

| **Example** | **8a** | **8b** | **8c** | **8d** | **8e** | **8f** |
|---|---|---|---|---|---|---|
| Ingredient | wt.-% | wt.-% | wt.-% | wt.-% | wt.-% | wt.-% |
| Piroctone olamine | 15 | 15 | 15 | 15 | 15 | 15 |
| Caprylyl glycol | 75 | 75 | 70 | 70 | 80 | 80 |
| Glycerin | 10 | - | - | - | - | - |
| Water | - | 10 | - | - | - | - |
| Ethanol | - | - | 15 | - | - | - |
| Propylene glycol | - | - | - | 15 | - | - |
| Sodium xylenesulfonate | - | - | - | - | 5 | - |
| Sodium cumenesulfonate | - | - | - | - | - | 5 |
| Total | 100 | 100 | 100 | 100 | 100 | 100 |

| **Example** | **8g** | **8h** | **8i** | **8j** | **8k** | **8l** |
|---|---|---|---|---|---|---|
| Ingredient | wt.-% | wt.-% | wt.-% | wt.-% | wt.-% | wt.-% |
| Piroctone olamine | 45 | 45 | 45 | 45 | 45 | 45 |
| Caprylyl glycol | 45 | 45 | 40 | 40 | 50 | 50 |
| Glycerin | 10 | - | - | - | - | - |
| Water | - | 10 | - | - | - | - |
| Ethanol | - | - | 15 | - | - | - |
| Propylene glycol | - | - | - | 15 | - | - |
| Sodium xylenesulfonate | - | - | - | - | 5 | - |
| Sodium cumenesulfonate | - | - | - | - | - | 5 |
| Total | 100 | 100 | 100 | 100 | 100 | 100 |

The blends can be prepared by mixing their ingredients. The blends are particularly useful for anti-dandruff applications and/or for preserving cosmetic compositions.

### Example 9

Cosmetic compositions (ingredients are given in wt%)

| **Example** | **9a** | **9b** | **9c** | **9d** | **9e** |
|---|---|---|---|---|---|
| Composition | Liquid soap | Body wash | Wet wipe lotion | Shampoo | Shampoo |
| Blend according to Example 3d | 0.5 | 0.5 | 1.0 | 1.0 | 1.0 |
| SLES | 10 | - | - | - | 9 |
| Cocamidopropylbetaine | 2 | 4.5 | - | - | 2 |
| Cocamide MEA | - | - | - | - | 1 |
| Sodium Cocoyl Glutamate | - | 2 | 1 | 2 | - |
| Sodium Cocoyl Glycinate | - | 2 | 1 | 2 | - |
| Sodium Cocoyl Isethionate | - | 2 | - | - | - |
| Cocoyl Methyl Glucamide | - | 3 | 2 | - | - |
| EGDS | - | 0.5 | - | - | 0.7 |
| PEG-7 Glyceryl Cocoate | - | - | 1 | - | - |
| Cocoyl Glucoside | - | - | - | 5 | - |
| Lauryl Glucoside | - | - | - | 5 | - |
| Acrylates Copolymer | - | - | - | 2 | - |
| Glycerin | - | - | - | 1 | 2 |
| Panthenol | - | - | - | 0.2 | 0.2 |
| Dimethicone | - | - | - | - | - |
| Polyquaternium 7 (PQ-7) | - | - | - | - | 0.6 |
| Guar Hydroxypropyltrimonium Chloride | - | - | - | - | 0.3 |
| Hydrolyzed Protein | - | - | - | - | 0.1 |
| N-fatty acyl-N-methyl cyclic glucamide | 1.5 | 2 | 1.0 | 1.5 | 2 |
| NaCl | 1.5 | 1.0 | 0.5 | 0.3 | 1.5 |
| Water | QSP | QSP | QSP | QSP | QSP |
| Total | 100 | 100 | 100 | 100 | 100 |

### Example 10

Cosmetic compositions (ingredients are given in wt%)

| **Example** | **10a** | **10b** | **10c** | **10d** |
|---|---|---|---|---|
| Composition | Body Lotion | Intensive hand and body lotion | Anti-ageing night cream | Baby cream |
| Blend according to Example 3e | 0.5 | 0.5 | 0.7 | 0.3 |
| Ethyhexyl Stearate | 7 | - | - | - |
| Decyl Oleate | 5 | - | - | - |
| Plantasens^{®} Natural Emulsifier HE 20 (Clariant), which is Cetearyl Glucoside (and) Sorbitan Olivate | 3 | - | - | - |
| Dimethicone | 2 | - | - | - |
| Glycerin | 3 | 3 | 7 | - |
| Xanthan Gum | 0.2 | - | - | - |
| Aristoflex^{®} AVC from Clariant (Ammonium Acryloyldimethyltaurate / VP Copolymer) | 0.5 | 1 | - | - |
| Polyglyceryl-2-Sesquiisostearate | - | 0.5 | - | 2 |
| Trilaureth-4 Phosphate | - | 2 | - | - |
| Mineral Oil (Paraffinum Liquidum) | - | 5 | - | - |
| Plantasens^{®} Refined Organic Babassu Butter from Clariant (Orbignya Oleifera Seed Oil) | - | 2 | - | - |
| Squalane | - | 2 | 12 | - |
| Macadamia Ternifolia Seed Oil (and) Crambe Abyssinica Seed Oil (and) Orbignya Oleifera Seed Oil | - | 2 | - | - |
| Silcare^{®} Silicone SEA from Clariant (Trideceth-9 PG-Amodimethicone and Trideceth-12) | - | 0.5 | - | - |
| Cetearyl Alcohol | - | 2 | - | - |
| Isopropyl Palmitate | - | 4 | - | - |
| Dow Corning^{®} 345 (Cyclopentasiloxane and Cyclohexasiloxane) | - | - | 10 | - |
| Paraffin Oil, low viscosity | - | - | 4 | 10 |
| Petrolatum | - | - | 4 | 15 |
| Cetyl Alcohol | - | - | 3 | - |
| Cutina^{®} GMS (Glyceryl Stearate) | - | - | 2.5 | - |
| PEG-40 Stearate | - | - | 3 | - |
| Cera Alba Wax | - | - | 2 | - |
| Mangifera Indica (Mango) Seed Butter | - | - | 2 | - |
| Abyssinian Oil (and) Phytosterols (and) Olea Europea (Olive) Oil Unsaponifiables | - | - | 0.5 | - |
| Sorbitan Tristearate | - | - | 0.3 | - |
| Ubiquinone | - | - | 0.05 | - |
| Aristoflex^{®} Velvet from Clariant (Polyacrylate Crosspolymer-11) | - | - | 0.3 | - |
| Hostacerin^{®} SFO from Clariant (Sunflower Seed Oil Sorbitol Esters) | - | - | - | 3.5 |
| Paracera^{®} M (Cera Microcristallina) | - | - | - | 2 |
| Beeswax | - | - | - | 1 |
| Magnesium Stearate | - | - | - | 1 |
| Talc | - | - | - | 10 |
| Zinc oxide | - | - | - | 10 |
| Allantoin (Clariant) | - | - | - | 0.3 |
| Extrapon Hamamelis | - | - | - | 2 |
| D-Panthenol | - | - | - | 2 |
| N-fatty acyl-N-methyl cyclic glucamide | 1.5 | 2 | 1.0 | 1.5 |
| Water | QSP | QSP | QSP | QSP |
| Total | 100 | 100 | 100 | 100 |

### Example 11

Cosmetic compositions (ingredients are given in wt%)

| **Example** | **11a** | **11b** | **11c** | **11d** | **11e** | **11f** |
|---|---|---|---|---|---|---|
| Composition | Soap formulation | Soap bar | Body wash | Shampoo | Wash cream | Body wash |
| Blend according to Example 3f | 0.5 | 2 | 0.8 | 1.0 | 0.6 | 0.5 |
| Sodium Lauryl Sulfate | - | - | 10 | - | - | - |
| Ammonium Lauryl Sulfate | - | - | - | 12 | - | - |
| Sodium Lauryl Ether Sulfate (SLES) | 15 | - | 2 | - | - | - |
| Cocamidopropylbetaine | 7 | - | 2 | 3 | - | 2 |
| Cocamide MEA | - | - | 1 | - | - | 2 |
| Lauric Acid | 0.5 | 2 | - | - | - | 10 |
| Myristic Acid | 1.5 | 2 | - | - | - | 10 |
| Stearic Acid | 0.5 | 2 | - | - | 10 | - |
| Palmitic Acid | - | 2 | - | - | - | 10 |
| Potassium Tallowate | - | 2 | - | - | - | - |
| Sodium Palm Kernelate | - | 20 | - | - | - | - |
| Sodium Cocoate | - | 60 | - | - | - | - |
| Lauryl Glucoside | - | - | - | 4 | - | - |
| Sodium Cocoyl Isethionate | - | - | - | - | 14 | - |
| Glycerin | - | 5 | - | - | - | 5 |
| Cetearyl Alcohol | 1.5 | - | - | - | 2 | - |
| Dimethicone | - | - | - | - | 0.1 | - |
| Polyquaternium 7 (PQ-7) | - | - | - | 0.2 | - | - |
| Guar Hydroxypropyltrimonium Chloride | - | - | 0.3 | - | - | - |
| Hydrolyzed Protein | - | - | - | 0.1 | - | - |
| N-fatty acyl-N-methyl cyclic glucamide | 1.5 | 2 | 1.0 | 1.5 | 1 | 0.8 |
| NaCl | 1.5 | - | 0.5 | 0.3 | - | 2.5 |

| Water | QSP | QSP | QSP | QSP | QSP | QSP |
|---|---|---|---|---|---|---|
| Total | 100 | 100 | 100 | 100 | 100 | 100 |

### Example 12

Cosmetic compositions according to Examples 9 to 11 where Ethylhexylglycerin is replaced by caprylyl glycol.

Cosmetic compositions according to Examples 9 to 11 where Ethylhexylglycerin is replaced by Garcinia Mangostana Peel Extract.

Cosmetic compositions according to Examples 9 to 11 where Ethylhexylglycerin is replaced by Orthosiphon Stamineus Leaf Extract.

### Example 13

Hair conditioner compositions comprising the following ingredients:

| **Example** | **13a** | **13b** | **13c** | **13d** | **13e** | **13f** |
|---|---|---|---|---|---|---|
| Ingredient | wt.-% | wt.-% | wt.-% | wt.-% | wt.-% | wt.-% |
| Blend according to Example 3d | 0.8 | 0.8 | 0.8 | 0.6 | 0.6 | 0.6 |
| Cetearyl Alcohol | 4.0 | 4.0 | 4.0 | 4.0 | 4.0 | 4.0 |
| CTAC | 2.0 | - | - | 1.0 | 1.0 | - |
| BTAC | - | 2.0 | - | 1.0 | - | 1.0 |
| BTMS | - | - | 2.0 | - | 1.0 | 1.0 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance^{®} Life: Clariant, INCI = Polyquaternium-116 und Butylene Glycol Genadvance^{®} Repair: Clariant, INCI = Quaternium-98 Genadvance^{®} Hydra: Clariant, INCI = Lauryl/Myristyl Polyricinoleate & Glycerin | | | | | | |

### Example 14

Hair conditioner compositions comprising the following ingredients:

| **Example** | **14a** | **14b** | **14c** | **14d** | **14e** | **14f** |
|---|---|---|---|---|---|---|
| Ingredient | wt.-% | wt.-% | wt.-% | wt.-% | wt.-% | wt.-% |
| Blend according to Example 3e | 0.8 | 0.8 | 0.5 | 0.8 | 0.7 | 0.6 |
| Cetearyl Alcohol | 4.0 | 4.0 | 4.0 | 4.0 | 4.0 | 4.0 |
| CTAC | 0.5 | 0.5 | 0.5 | - | - | - |
| BTAC | - | - | - | 0.5 | 0.5 | 0.5 |
| Genadvance^{®} Life | 1.5 | - | - | 1.5 | - | - |
| Genadvance^{®} Repair | - | 1.5 | - | - | 1.5 | - |
| Genadvance^{®} Hydra | - | - | 1.5 | - | - | 1.5 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance^{®} Life: Clariant, INCI = Polyquaternium-116 und Butylene Glycol Genadvance^{®} Repair: Clariant, INCI = Quaternium-98 Genadvance^{®} Hydra: Clariant, INCI = Lauryl/Myristyl Polyricinoleate & Glycerin | | | | | | |

### Example 15

Hair conditioner compositions comprising the following ingredients:

| **Example** | **15a** | **15b** | **15c** | **15d** | **15e** | **15f** |
|---|---|---|---|---|---|---|
| Ingredient | wt.-% | wt.-% | wt.-% | wt.-% | wt.-% | wt.-% |
| Blend according to Example 3f | 0.5 | 0.5 | 0.3 | 0.5 | 0.5 | 0.6 |
| Cetearyl Alcohol | 4.0 | 4.0 | 4.0 | 4.0 | 4.0 | 4.0 |
| CTAC | 0.2 | 0.2 | 0.2 | - | - | - |
| BTAC | - | - | - | 0.2 | 0.2 | 0.2 |
| Genadvance^{®} Life | 1.8 | - | - | 1.8 | - | - |
| Genadvance^{®} Repair | - | 1.8 | - | - | 1.8 | - |
| Genadvance^{®} Hydra | - | - | 1.8 | - | - | 1.8 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance^{®} Life: Clariant, INCI = Polyquaternium-116 und Butylene Glycol Genadvance^{®} Repair: Clariant, INCI = Quaternium-98 Genadvance^{®} Hydra: Clariant, INCI = Lauryl/Myristyl Polyricinoleate & Glycerin | | | | | | |

### Example 16

Hair conditioner compositions according to Examples 13 to 15 where Ethylhexylglycerin is replaced by caprylyl glycol.

Hair conditioner compositions according to Examples 13 to 15 where Ethylhexylglycerin is replaced by Garcinia Mangostana Peel Extract.

Hair conditioner compositions according to Examples 13 to 15 where Ethylhexylglycerin is replaced by Orthosiphon Stamineus Leaf Extract.

### Example 17

Shampoo compositions comprising the following ingredients:

| **Example** | **17a** | **17b** | **17c** | **17d** |
|---|---|---|---|---|
| Ingredient | wt% | wt% | wt% | wt% |
| Aqua | To 100 | To 100 | To 100 | To 100 |
| Blend according to Example 3c | 1.5 | - | - | - |
| Blend according to Example 4e | - | 1.5 | - | - |
| Blend according to Example 5d | - | - | 2.0 | - |
| Blend according to Example 6e | - | - | - | 2.0 |
| sodium laureth sulfate | 8 | 8 | 8 | 8 |
| sodium lauryl sulfate | 8 | 8 | 8 | 8 |
| cocamide MIPA | 4 | 4 | 4 | 4 |
| glycerin | 3.5 | 3.5 | 3.5 | 3.5 |
| glycol distearate | 3 | 3 | 3 | 3 |
| sodium chloride | 2.5 | 2.5 | 2.5 | 2.5 |
| dimethicone | 2 | 2 | 2 | 2 |
| PPG-5-ceteth-20 | 1.5 | 1.5 | 1.5 | 1.5 |
| Hair conditioning agent | 1 | 1 | 1 | 1 |
| carbomer | 0.4 | 0.4 | 0.4 | 0.4 |
| niacinamide | 0.2 | 0.2 | 0.2 | 0.2 |
| pyridoxine HCI | 0.2 | 0.2 | 0.2 | 0.2 |
| guar hydroxypropyltrimonium chloride | 0.2 | 0.2 | 0.2 | 0.2 |
| hydrolyzed soy protein | 0.2 | 0.2 | 0.2 | 0.2 |
| methyl cocoate | 0.15 | 0.15 | 0.15 | 0.15 |
| sodium cocoate | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 18

Shampoo compositions comprising the following ingredients:

| **Example** | **18a** | **18b** | **18c** | **18d** |
|---|---|---|---|---|
| Ingredient | wt% | wt% | wt% | wt% |
| Aqua | To 100 | To 100 | To 100 | To 100 |
| Blend according to Example 3d | 1.5 | - | - | - |
| Blend according to Example 4f | - | 1.5 | - | - |
| Blend according to Example 5e | - | - | 1.5 | - |
| Blend according to Example 6f | - | - | - | 1.5 |
| sodium laureth sulfate | 14 | 14 | 14 | 14 |
| coco-betaine | 4 | 4 | 4 | 4 |
| glycerin | 3 | 3 | 3 | 3 |
| glycol distearate | 2.5 | 2.5 | 2.5 | 2.5 |
| dimethicone | 2 | 2 | 2 | 2 |
| Hair conditioning agent | 1.5 | 1.5 | 1.5 | 1.5 |
| niacinamide | 0.5 | 0.5 | 0.5 | 0.5 |
| coconut oil | 0.5 | 0.5 | 0.5 | 0.5 |
| sodium chloride | 0.5 | 0.5 | 0.5 | 0.5 |
| olive fruit oil | 0.4 | 0.4 | 0.4 | 0.4 |
| PPG-5-ceteth-20 | 0.3 | 0.3 | 0.3 | 0.3 |
| trideceth-6 | 0.25 | 0.25 | 0.25 | 0.25 |
| polyquaternium-6 | 0.25 | 0.25 | 0.25 | 0.25 |
| amodimethicone | 0.2 | 0.2 | 0.2 | 0.2 |
| carbomer | 0.2 | 0.2 | 0.2 | 0.2 |
| cetrimonium chloride | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 19

Shampoo compositions comprising the following ingredients:

| **Example** | **19a** | **19b** | **19c** | **19d** |
|---|---|---|---|---|
| Ingredient | wt% | wt% | wt% | wt% |
| Aqua | To 100 | To 100 | To 100 | To 100 |
| Blend according to Example 3e | 1.5 | - | - | - |
| Blend according to Example 4c | - | 1.5 | - | - |
| Blend according to Example 5f | - | - | 1.0 | - |
| Blend according to Example 6d | - | - | - | 1.0 |
| ammonium lauryl sulfate | 16 | 16 | 16 | 16 |
| cocoamidopropyl betaine | 5 | 5 | 5 | 5 |
| sodium chloride | 2.5 | 2.5 | 2.5 | 2.5 |
| niacinamide | 2 | 2 | 2 | 2 |
| Hair conditioning agent | 1 | 1 | 1 | 1 |
| hydroxypropyl guar hydroxypropyltrimonium chloride | 0.8 | 0.8 | 0.8 | 0.8 |
| propylene glycol | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 20

Shampoo compositions comprising the following ingredients:

| **Example** | **20a** | **20b** | **20c** | **20d** |
|---|---|---|---|---|
| Ingredient | wt% | wt% | wt% | wt% |
| Aqua | To 100 | To 100 | To 100 | To 100 |
| Blend according to Example 3f | 1.0 | - | - | - |
| Blend according to Example 4d | - | 1.5 | - | - |
| Blend according to Example 5d | - | - | 1.0 | - |
| Blend according to Example 6e | - | - | - | 1.0 |
| sodium laureth sulfate | 16 | 16 | 16 | 16 |
| glycol distearate | 4 | 4 | 4 | 4 |
| coco-betaine | 2.5 | 2.5 | 2.5 | 2.5 |
| glycerin | 2 | 2 | 2 | 2 |
| sodium chloride | 1.5 | 1.5 | 1.5 | 1.5 |
| dimethicone | 1.5 | 1.5 | 1.5 | 1.5 |
| Hair conditioning agent | 1.5 | 1.5 | 1.5 | 1.5 |
| niacinamide | 1 | 1 | 1 | 1 |
| guar hydroxypropyltrimonium chloride | 0.8 | 0.8 | 0.8 | 0.8 |
| cocamide MIPA | 0.5 | 0.5 | 0.5 | 0.5 |
| PPG-5-ceteth-20 | 0.2 | 0.2 | 0.2 | 0.2 |
| fumaric acid | 0.15 | 0.15 | 0.15 | 0.15 |
| carbomer | 0.15 | 0.15 | 0.15 | 0.15 |
| pyridoxine HCI | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 21

Shampoo compositions comprising the following ingredients:

| **Example** | **21a** | **21b** | **21c** | **21d** |
|---|---|---|---|---|
| Ingredient | wt% | wt% | wt% | wt% |
| Aqua | To 100 | To 100 | To 100 | To 100 |
| Blend according to Example 3c | 1.0 | - | - | - |
| Blend according to Example 4e | - | 1.0 | - | - |
| Blend according to Example 5e | - | - | 1.5 | - |
| Blend according to Example 6f | - | - | - | 1.0 |
| sodium laureth sulfate | 16 | 16 | 16 | 16 |
| coco-betaine | 4 | 4 | 4 | 4 |
| niacinamide | 2 | 2 | 2 | 2 |
| Ribes nigrum oil | 1.5 | 1.5 | 1.5 | 1.5 |
| cocamide MIPA | 1.5 | 1.5 | 1.5 | 1.5 |
| sodium chloride | 1.5 | 1.5 | 1.5 | 1.5 |
| Hair conditioning agent | 1.2 | 1.2 | 1.2 | 1.2 |
| sodium cocoate | 0.8 | 0.8 | 0.8 | 0.8 |
| cocamide MIPA | 0.5 | 0.5 | 0.5 | 0.5 |
| Olea europaea oil (olive) fruit oil | 0.3 | 0.3 | 0.3 | 0.3 |
| PEG-55 propylene glycol oleate | 0.3 | 0.3 | 0.3 | 0.3 |
| PEG-60 hydrogenated castor oil | 0.3 | 0.3 | 0.3 | 0.3 |
| polyquaternium-7 | 0.2 | 0.2 | 0.2 | 0.2 |
| amodimethicone | 0.15 | 0.15 | 0.15 | 0.15 |
| propylene glycol | 0.1 | 0.1 | 0.1 | 0.1 |
| Butyrospermum parkii butter | 0.1 | 0.1 | 0.1 | 0.1 |
| laureth-5 carboxylic acid | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 22

Shampoo compositions comprising the following ingredients:

| **Example** | **22a** | **22b** | **22c** | **22d** |
|---|---|---|---|---|
| Ingredient | wt% | wt% | wt% | wt% |
| Aqua | To 100 | To 100 | To 100 | To 100 |
| Blend according to Example 3d | 1.5 | - | - | - |
| Blend according to Example 4f | - | 1.0 | - | - |
| Blend according to Example 5f | - | - | 1.5 | - |
| Blend according to Example 6d | - | - | - | 1.0 |
| sodium laureth sulfate | 10 | 10 | 10 | 10 |
| disodium cocamphodiacetate | 5 | 5 | 5 | 5 |
| glycerin | 3 | 3 | 3 | 3 |
| glycol distearate | 2 | 2 | 2 | 2 |
| sodium chloride | 1.5 | 1.5 | 1.5 | 1.5 |
| Hair conditioning agent | 1.5 | 1.5 | 1.5 | 1.5 |
| cocamide MEA | 1 | 1 | 1 | 1 |
| polyquaternium-10 | 1 | 1 | 1 | 1 |
| carbomer | 0.3 | 0.3 | 0.3 | 0.3 |
| citric acid | As needed, pH 4.5 - 6.5 | | | |
| sodium hydroxide | As needed, pH 4.5 - 6.5 | | | |
| Colorant | As needed | | | |

### Example 23

Shampoo compositions comprising the following ingredients:

| **Example** | **23a** | **23b** | **23c** | **23d** |
|---|---|---|---|---|
| Ingredient | wt% | wt% | wt% | wt% |
| Aqua | To 100 | To 100 | To 100 | To 100 |
| Blend according to Example 3e | 1.5 | - | - | - |
| Blend according to Example 4c | - | 1.0 | - | - |
| Blend according to Example 5d | - | - | 1.0 | - |
| Blend according to Example 6e | - | - | - | 1.0 |
| sodium laureth sulfate | 12 | 12 | 12 | 12 |
| coco-betaine | 3 | 3 | 3 | 3 |
| cocamide MIPA | 2 | 2 | 2 | 2 |
| sodium chloride | 1.8 | 1.8 | 1.8 | 1.8 |
| Olea europaea (olive) fruit oil | 0.5 | 0.5 | 0.5 | 0.5 |
| Hair conditioning agent | 0.5 | 0.5 | 0.5 | 0.5 |
| PPG-5-ceteth-20 | 0.4 | 0.4 | 0.4 | 0.4 |
| PEG-55 propylene glycol oleate | 0.4 | 0.4 | 0.4 | 0.4 |
| PEG-60 hydrogenated castor oil | 0.25 | 0.25 | 0.25 | 0.25 |
| polyquaternium-10 | 0.25 | 0.25 | 0.25 | 0.25 |
| amodimethicone | 0.15 | 0.15 | 0.15 | 0.15 |
| propylene glycol | 0.15 | 0.15 | 0.15 | 0.15 |
| laureth-5 | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 24

Shampoo compositions comprising the following ingredients:

| **Example** | **24a** | **24b** | **24c** | **24d** |
|---|---|---|---|---|
| Ingredient | wt% | wt% | wt% | wt% |
| Aqua | To 100 | To 100 | To 100 | To 100 |
| Blend according to Example 3f | 1.5 | - | - | - |
| Blend according to Example 4d | - | 1.0 | - | - |
| Blend according to Example 5e | - | - | 1.0 | - |
| Blend according to Example 6f | - | - | - | 1.0 |
| ammonium lauryl sulfate | 15 | 15 | 15 | 15 |
| cocamidopropyl betaine | 3 | 3 | 3 | 3 |
| sodium chloride | 2 | 2 | 2 | 2 |
| propylene glycol | 1.8 | 1.8 | 1.8 | 1.8 |
| hydroxypropyl guar hydroxypropyltrimonium chloride | 1.5 | 1.5 | 1.5 | 1.5 |
| Hair conditioning agent | 1 | 1 | 1 | 1 |
| citric acid | As needed, pH 4.5 - 6.5 | | | |
| sodium hydroxide | As needed, pH 4.5 - 6.5 | | | |
| Colorant | As needed | | | |

### Example 25

Shampoo compositions comprising the following ingredients:

| **Example** | **25a** | **25b** | **25c** | **25d** |
|---|---|---|---|---|
| Ingredient | wt% | wt% | wt% | wt% |
| Aqua | To 100 | To 100 | To 100 | To 100 |
| Blend according to Example 3c | 1.5 | - | - | - |
| Blend according to Example 4e | - | 1.0 | - | - |
| Blend according to Example 5f | - | - | 1.0 | - |
| Blend according to Example 6d | - | - | - | 1.0 |
| sodium cocoyl isethionate | 4 | 4 | 4 | 4 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.5 | 2.5 | 2.5 | 2.5 |
| glycol distearate | 2 | 2 | 2 | 2 |
| glycereth-26 | 1.8 | 1.8 | 1.8 | 1.8 |
| decyl glucoside | 1.5 | 1.5 | 1.5 | 1.5 |
| hydrogenated coconut acid | 1.4 | 1.4 | 1.4 | 1.4 |
| PPG-5-ceteth-20 | 1.3 | 1.3 | 1.3 | 1.3 |
| divinyldimethicone/dimethicone copolymer | 1.2 | 1.2 | 1.2 | 1.2 |
| sodium chloride | 1.1 | 1.1 | 1.1 | 1.1 |
| amodimethicone | 1 | 1 | 1 | 1 |
| Hair conditioning agent | 1 | 1 | 1 | 1 |
| polyquaternium-7 | 1 | 1 | 1 | 1 |
| polyquaternium-10 | 0.7 | 0.7 | 0.7 | 0.7 |
| sodium isethionate | 0.5 | 0.5 | 0.5 | 0.5 |
| PEG-55 propylene glycol oleate | 0.4 | 0.4 | 0.4 | 0.4 |
| propylene glycol | 0.4 | 0.4 | 0.4 | 0.4 |
| carbomer | 0.4 | 0.4 | 0.4 | 0.4 |
| C11-15 pareth-7 | 0.3 | 0.3 | 0.3 | 0.3 |
| glycerin | 0.3 | 0.3 | 0.3 | 0.3 |
| trideceth-12 | 0.25 | 0.25 | 0.25 | 0.25 |
| laureth-9 | 0.2 | 0.2 | 0.2 | 0.2 |
| hydroxypropyltrimonium hydrolyzed wheat protein | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 26

Shampoo compositions comprising the following ingredients:

| **Example** | **26a** | **26b** | **26c** | **26d** |
|---|---|---|---|---|
| Ingredient | wt% | wt% | wt% | wt% |
| Aqua | To 100 | To 100 | To 100 | To 100 |
| Blend according to Example 3d | 1.5 | - | - | - |
| Blend according to Example 4f | - | 1.5 | - | - |
| Blend according to Example 5d | - | - | 1.5 | - |
| Blend according to Example 6e | - | - | - | 1.5 |
| sodium methyl cocoyl taurate | 5 | 5 | 5 | 5 |
| laureth-5 carboxylic acid | 4 | 4 | 4 | 4 |
| sodium chloride | 2 | 2 | 2 | 2 |
| cocamidopropyl betaine | 2 | 2 | 2 | 2 |
| glycerin | 1.8 | 1.8 | 1.8 | 1.8 |
| glycol distearate | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5 ceteth-20 | 0.5 | 0.5 | 0.5 | 0.5 |
| sodium lauroyl glutamate | 0.4 | 0.4 | 0.4 | 0.4 |
| propylene glycol | 0.2 | 0.2 | 0.2 | 0.2 |
| PEG-55 propylene glycol oleate | 0.15 | 0.15 | 0.15 | 0.15 |
| Argania spinosa kernel oil | 0.1 | 0.1 | 0.1 | 0.1 |
| citric acid, lactic acid | As needed, pH 4.5 - 6.5 | | | |
| sodium hydroxide | As needed, pH 4.5 - 6.5 | | | |
| Colorant | As needed | | | |

### Example 27

Shampoo compositions comprising the following ingredients:

| **Example** | **27a** | **27b** | **27c** | **27d** |
|---|---|---|---|---|
| Ingredient | wt% | wt% | wt% | wt% |
| Aqua | To 100 | To 100 | To 100 | To 100 |
| Blend according to Example 3e | 1.0 | - | - | - |
| Blend according to Example 4c | - | 1.0 | - | - |
| Blend according to Example 5e | - | - | 1.0 | - |
| Blend according to Example 6f | - | - | - | 1.0 |
| sodium laureth sulfate | 10 | 10 | 10 | 10 |
| disodium cocoamphodiacetate | 4 | 4 | 4 | 4 |
| glycol distearate | 2 | 2 | 2 | 2 |
| sodium chloride | 1.5 | 1.5 | 1.5 | 1.5 |
| Hair conditioning agent | 1.3 | 1.3 | 1.3 | 1.3 |
| carbomer | 0.4 | 0.4 | 0.4 | 0.4 |
| diaminopyrimidine oxide | 0.3 | 0.3 | 0.3 | 0.3 |
| disodium ricinoleamido MEA sulfosuccinate | 0.3 | 0.3 | 0.3 | 0.3 |
| propylene glycol | 0.2 | 0.2 | 0.2 | 0.2 |
| panthenol | 0.1 | 0.1 | 0.1 | 0.1 |
| polyquaternium-10 | 0.1 | 0.1 | 0.1 | 0.1 |
| citric acid | As needed, pH 4.5 - 6.5 | | | |
| sodium hydroxide, ammonium hydroxide | As needed, pH 4.5 - 6.5 | | | |
| Colorant | As needed | | | |

### Example 28

Shampoo compositions comprising the following ingredients:

| **Example** | **28a** | **28b** | **28c** | **28d** |
|---|---|---|---|---|
| Ingredient | wt% | wt% | wt% | wt% |
| Aqua | To 100 | To 100 | To 100 | To 100 |
| Blend according to Example 3f | 1.5 | - | - | - |
| Blend according to Example 4d | - | 1.0 | - | - |
| Blend according to Example 5f | - | - | 1.5 | - |
| Blend according to Example 6d | - | - | - | 1.5 |
| sodium cocoyl isethionate | 4.5 | 4.5 | 4.5 | 4.5 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.3 | 2.3 | 2.3 | 2.3 |
| glycol distearate | 2 | 2 | 2 | 2 |
| glycereth-26 | 1.8 | 1.8 | 1.8 | 1.8 |
| decyl glucoside | 1.7 | 1.7 | 1.7 | 1.7 |
| hydrogenated coconut acid | 1.5 | 1.5 | 1.5 | 1.5 |
| PPG-5-ceteth-20 | 1.5 | 1.5 | 1.5 | 1.5 |
| PEG-55 propylene glycol oleate | 1.5 | 1.5 | 1.5 | 1.5 |
| propylene glycol | 1.5 | 1.5 | 1.5 | 1.5 |
| sodium chloride | 1.5 | 1.5 | 1.5 | 1.5 |
| divinyldimethicone/dimethicone copolymer | 1 | 1 | 1 | 1 |
| polyquaternium-7 | 1 | 1 | 1 | 1 |
| amodimethicone | 1 | 1 | 1 | 1 |
| Hair conditioning agent | 1 | 1 | 1 | 1 |
| polyquaternium-10 | 0.8 | 0.8 | 0.8 | 0.8 |
| sodium isethionate | 0.6 | 0.6 | 0.6 | 0.6 |
| carbomer | 0.6 | 0.6 | 0.6 | 0.6 |
| C11-15 pareth-7 | 0.4 | 0.4 | 0.4 | 0.4 |
| glycerin | 0.3 | 0.3 | 0.3 | 0.3 |
| laureth-9 | 0.2 | 0.2 | 0.2 | 0.2 |
| citric acid | As needed, pH 4.5 - 6.5 | | | |
| sodium hydroxide | As needed, pH 4.5 - 6.5 | | | |
| Colorant | As needed | | | |

### Example 29

Shampoo compositions comprising the following ingredients:

| **Example** | **29a** | **29b** | **29c** | **29d** |
|---|---|---|---|---|
| Ingredient | wt% | wt% | wt% | wt% |
| Aqua | To 100 | To 100 | To 100 | To 100 |
| Blend according to Example 3c | 1.0 | - | - | - |
| Blend according to Example 4e | - | 1.0 | - | - |
| Blend according to Example 5d | - | - | 1.5 | - |
| Blend according to Example 6e | - | - | - | 1.5 |
| cocamidopropyl betaine | 5 | 5 | 5 | 5 |
| sodium lauryl sulfoacetate | 4 | 4 | 4 | 4 |
| disodium laureth sulfosuccinate | 3.5 | 3.5 | 3.5 | 3.5 |
| sodium lauroyl sarcosinate | 3 | 3 | 3 | 3 |
| glycol distearate | 2 | 2 | 2 | 2 |
| sodium chloride | 1.7 | 1.7 | 1.7 | 1.7 |
| decyl glucoside | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5-ceteth-20 | 1 | 1 | 1 | 1 |
| coco-betaine | 0.8 | 0.8 | 0.8 | 0.8 |
| PEG-55 propylene glycol oleate | 0.8 | 0.8 | 0.8 | 0.8 |
| propylene glycol | 0.5 | 0.5 | 0.5 | 0.5 |
| carbomer | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

### Example 30

Shampoo compositions comprising the following ingredients:

| **Example** | **30a** | **30b** | **30c** | **30d** |
|---|---|---|---|---|
| Ingredient | wt% | wt% | wt% | wt% |
| Aqua | To 100 | To 100 | To 100 | To 100 |
| Blend according to Example 3d | 1.5 | - | - | - |
| Blend according to Example 4f | - | 1.0 | - | - |
| Blend according to Example 5e | - | - | 1.5 | - |
| Blend according to Example 6f | - | - | - | 1.5 |
| sodium laureth sulfate | 15 | 15 | 15 | 15 |
| coco-betaine | 3 | 3 | 3 | 3 |
| glycol distearate | 2.5 | 2.5 | 2.5 | 2.5 |
| sodium chloride | 2 | 2 | 2 | 2 |
| PPG-5-ceteth-20 | 1.8 | 1.8 | 1.8 | 1.8 |
| Hair conditioning agent | 1.5 | 1.5 | 1.5 | 1.5 |
| octyldodecanol | 1 | 1 | 1 | 1 |
| carbomer | 1 | 1 | 1 | 1 |
| guar hydroxypropyltrimonium chloride | 1 | 1 | 1 | 1 |
| sodium hyaluronate | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

### Example 31

Cosmetic compositions according to Examples 9 to 30 where piroctone olamine and the booster are added separately, i.e. not in the form of a blend.

## Claims

1. Use of a booster to enhance the anti-dandruff activity of piroctone olamine or to enhance the preservative activity of piroctone olamine, wherein the booster is selected from alcohols having 2 to 5 hydroxy groups and plant extracts.

2. Use according to claim 1, wherein the booster is selected from alcohols having 2 to 5 hydroxy groups, preferably diols, more preferably diols having 2 to 20 carbon atoms, particularly preferably diols having 4 to 12 carbon atoms.

3. Use according to claim 2, wherein the diols are ethylhexylglycerin, caprylyl glycol, caprylyl glyceryl ether, 1,3-octanediol, 1,2-hexanediol, pentylene glycol, 1,2-decanediol, 4-n-butyl resorcinol, or mixtures thereof.

4. Use according to claim 1, wherein the booster is selected from plant extracts.

5. Blend comprising piroctone olamine and a booster selected from alcohols having 2 to 5 hydroxy groups and plant extracts.

6. Blend according to claim 5, wherein the blend comprises
from 2 to 70 wt.-%, preferably from 5 to 60 wt.-%, particularly preferably from 10 to 55 wt.-% of piroctone olamine, based on the total weight of the blend, and
from 30 to 98 wt.-%, preferably from 40 to 95 wt.-%, particularly preferably from 45 to 90 wt.-% of the booster, based on the total weight of the blend.

7. Blend according to claim 5 or 6, wherein the booster is selected from alcohols having 2 to 5 hydroxy groups, preferably diols, more preferably diols having 2 to 20 carbon atoms, particularly preferably diols having 4 to 12 carbon atoms.

8. Blend according to claim 7, wherein the diols are ethylhexylglycerin, caprylyl glycol, caprylyl glyceryl ether, 1,3-octanediol, 1,2-hexanediol, pentylene glycol, 1,2-decanediol, 4-n-butyl resorcinol, or mixtures thereof.

9. Blend according to claim 5 or 6, wherein the booster is selected from plant extracts.

10. Cosmetic composition, preferably shampoo composition, comprising piroctone olamine and a booster selected from alcohols having 2 to 5 hydroxy groups and plant extracts.

11. Cosmetic composition according to claim 10, wherein the cosmetic composition comprises
from 0.02 to 7 wt.-%, preferably from 0.1 to 2.0 wt.-%, particularly preferably from 0.1 to 1.0 wt.-% of piroctone olamine, based on the total weight of the cosmetic composition, and
from 0.02 to 7 wt.-%, preferably from 0.1 to 2.0 wt.-%, particularly preferably from 0.1 to 1.0 wt.-% of the booster, based on the total weight of the cosmetic composition.

12. Cosmetic composition according to claim 10 or 11, wherein the booster is selected from alcohols having 2 to 5 hydroxy groups, preferably diols, more preferably diols having 2 to 20 carbon atoms, particularly preferably diols having 4 to 12 carbon atoms.

13. Cosmetic composition according to claim 12, wherein the diols are ethylhexylglycerin, caprylyl glycol, caprylyl glyceryl ether, 1,3-octanediol, 1,2-hexanediol, pentylene glycol, 1,2-decanediol, 4-n-butyl resorcinol, or mixtures thereof.

14. Cosmetic composition according to claim 10 or 11, wherein the booster is selected from plant extracts.

15. Use of piroctone olamine and a booster as an anti-dandruff system or as a preservative system, wherein the booster is selected from alcohols having 2 to 5 hydroxy groups and plant extracts.
